# EUROPÄISCHE PATENTSCHRIFT

(11) **EP 1 414 505 B1**
(45) Veröffentlichungstag und Bekanntmachung des Hinweises auf die Patenterteilung: **03.01.2007**
(21) Anmeldenummer: 02742641.0
(22) Anmeldetag: 22.07.2002
(51) Int. Cl.: A61M 5/00, A61M 5/315

(54) **VERABREICHUNGSGERÄT MIT DOSIERVORRICHTUNG**
ADMINISTRATION APPLIANCE COMPRISING A DOSAGE DEVICE
APPAREIL D'ADMINISTRATION COMPORTANT UN DISPOSITIF DE DOSAGE

(30) Priorität: 30.07.2001 DE 20112501 U; 21.12.2001 DE 10163326
(43) Veröffentlichungstag der Anmeldung: 06.05.2004
(73) Patentinhaber: TecPharma Licensing AG, 3401 Burgdorf (CH)
(72) Erfinder: KIRCHHOFER, Fritz, CH-3454 Sumiswald (CH); GRAF, Roney, CH-3400 Burgdorf (CH)
(86) Internationale Anmeldenummer: PCT/CH2002/000410
(87) Internationale Veröffentlichungsnummer: WO 2003/011371

(56) Entgegenhaltungen:
- EP-A- 0 295 075
- EP-A- 0 498 737
- EP-A- 0 594 349
- EP-A- 1 095 668
- WO-A-97/17095

## Beschreibung

Die Erfindung betrifft Verabreichungsgeräte, vorzugsweise Injektionsgeräte, die eine Auswahl einer auszuschüttenden Produktdosis erlauben und bevorzugt für medizinische, therapeutische, diagnostische, pharmazeutische oder kosmetische Anwendungen vorgesehen sind. Bevorzugte Beispiele von Injektionsgeräten sind Injektionspens, insbesondere Semidisposable Pens. Ein Verabreichungsgerät nach der Erfindung kann beispielsweise auch ein Inhalationsgerät oder ein Gerät zur dosierten Abgabe eines oral einzunehmenden Produkts sein.

Verabreichungsgeräte sollen im Allgemeinen handlich und daher klein sein, andererseits aber eine möglichst hohe Funktionalität aufweisen. Dem ersten Aspekt werden sogenannte Injektionspens, die ihrer schlanken Form wegen so bezeichnet werden, ohne weiteres gerecht. Ein wesentlicher Aspekt im Hinblick auf die Funktionalität ist die Fähigkeit der freien Auswahl der mit einer Injektion zu injizierenden Produktdosis. Die Möglichkeit der Produktdosisauswahl ist insbesondere in solchen Anwendungen von Vorteil, in denen ein Verwender sich das zu injizierende Produkt selbst verabreicht, wie dies beispielsweise in der Diabetestherapie oder in der Hormonverabreichung üblich ist, um nur zwei bevorzugte Anwendungsbeispiele zu nennen. Mit der Möglichkeit der flexiblen Produktdosisauswahl einher geht jedoch ein entsprechender technischer Aufwand, der nicht nur den Preis erhöht, sondern auch die betreffenden Geräte vergrößert.

Ein Injektionspen mit Produktdosisauswahl wird in der US-PS 4,973,318 beschrieben. Der Pen weist eine Kolbenstange auf, die von einer Gewindestange gebildet wird, und dazu dient, einen Kolben in einer Produktampulle zu verschieben und dadurch das Produkt auszuschütten. Der Pen weist eine vordere Gehäusehülse und eine hintere Gehäusehülse auf, die um eine gemeinsame Längsachse relativ zueinander verdrehbar sind. Durch die Relatiwerdrehung der beiden Gehäusehülsen wird die Produktdosis ausgewählt. Die Kolbenstange steht mit einer Gewindemutter in einem Gewindeeingriff. Die Gewindemutter bildet einen vorderen Abschnitt eines hülsenförmigen Dosier- und Betätigungselements. Dieses Dosier- und Betätigungselement ragt an einem hinteren Ende in die hintere Gehäusehülse ein und ist mit der hinteren Gehäusehülse verdrehgesichert verbunden, aber relativ zu der hinteren Gehäusehülse in Längsrichtung der Kolbenstange hin und her verschiebbar. Wird die hintere Gehäusehülse zum Zwecke der Dosisauswahl gedreht, so wird das Dosier- und Betätigungselement zwangsweise mitgedreht. Da die Kolbenstange jedoch mit der vorderen Gehäusehülse verdrehgesichert verbunden ist, wird das Dosier- und Betätigungselement aufgrund der Drehbewegung weiter aus der hinteren Gehäusehülse nach hinten hinaus bewegt. Hierbei wird die Gesamtlänge der Anordnung aus Kolbenstange und Dosier- und Betätigungselement vergrößert und ein lichter Abstand zwischen dem vorderen Ende des Dosier- und Betätigungselements und einer Anschlagfläche des Gehäuses vergrößert. Dieser lichte Abstand entspricht dem maximal möglichen Hub, wenn das Dosier- und Betätigungselement zusammen mit der Kolbenstange zum Zwecke der Produktausschüttung in Richtung auf ein vorderes Ende des Pens vorgeschoben wird. Wegen dieser recht einfachen Dosiermechanik entspricht der Hub der Ausschüttbewegung der jeweils eingestellten Produktdosis und ist somit variabel.

Aus der WO 97/17096 ist ein Injektionspen bekannt, der unabhängig von der ausgewählten Produktdosis stets den gleichen Ausschütthub aufweist. Die Kolbenstange ist ebenfalls als Gewindestange ausgebildet und verdrehgesichert mit dem Gehäuse des Pens verbunden. Eine Dosiseinstellmutter steht mit der Kolbenstange in einem Gewindeeingriff. Die Kolbenstange ragt in ein hülsenförmiges Dosier- und Betätigungselement. Das Dosier- und Betätigungselement und die Dosiseinstellmutter dieses Pens sind separate Teile. Das Dosier- und Betätigungselement ist mit der Dosiseinstellmutter verdrehgesichert, aber in Längsrichtung der Kolbenstange verschieblich verbunden. Für diesen Eingriff umgibt das Dosier- und Betätigungselement die Dosiseinstellmutter. Für die Auswahl der Produktdosis wird das Dosier- und Betätigungselement um die Längsachse verdreht. Die Dosiseinstellmutter wird hierbei mitgedreht. Aufgrund des Gewindeeingriffs mit der Kolbenstange, der Linearführung der Dosiseinstellmutter an dem Gehäuse und der Sperrung der Kolbenstange gegen eine Bewegung entgegen der Vorschubrichtung wird die Dosiseinstellmutter bei der Drehbewegung entlang der Kolbenstange nach hinten und damit tiefer in das Dosier- und Betätigungselement hinein bewegt. Zwischen dem vorderen Ende der Dosiseinstellmutter und einem in Vorschubrichtung gegenüberliegenden Anschlag des Gehäuses ergibt sich ein lichter Abstand, der dem bei der Ausschüttbewegung von der Kolbenstange und der Dosiseinstellmutter gemeinsam zurücklegbaren Weg und damit der Produktdosis entspricht. Zur Bildung der verdrehgesicherten Verbindung zwischen dem Dosier-und Betätigungselement und der Dosiseinstellmutter ist eine Überlappung dieser beiden Teile entlang der Längsachse erforderlich, wodurch der Durchmesser des Pens vergrößert wird. Bei Verwendung des Dosiermechanismus in einem Semidisposable Injektionspen wird der Zusammenbau der Teile solch eines Pens erschwert, falls die Dosiseinstellmutter einerseits und das Dosier- und Betätigungselement andererseits je Bestandteil von Penteilen sind, die miteinander verbunden werden müssen.

Aus der EP 0 594 349 A1 ist ein Verabreichungsgerät gemäß dem Oberbegriff des Patentanspruchs 1 bekannt. Eine Kolbenstange mit einem Gewinde ist vorgesehen, wobei mittels einer Drehbewegung ein Kolben voranbewegt wird, der auf ein Reservoir einwirkt, um ein Produkt aus dem Verabreichungsgerät auszuschütten. Da keine Linearbewegung für die Kolbenstange vorgesehen ist, sondern eine Drehbewegung, ist es möglich, dass die mit einem Außengewinde versehene Kolbenstange am Ende ihrer für den Kolben gedachten Vortriebsbewegung die Vortriebsbewegung in eine umgekehrte Bewegung wandelt, so dass das auszuschüttende Produkt wieder zurückgesaugt werden könnte. Um diesem Nachteil entgegenzuwirken, wird eine Sperre vorgesehen, die eine Umkehr der Vortriebsbewegung in eine Rückwärtsbewegung verhindern soll.

Es ist eine Aufgabe der Erfindung, ein Verabreichungsgerät mit Produktdosisauswahl zu schaffen, das schlank und preiswert ist und einen konstanten Ausschütthub ermöglicht.

Eine andere Aufgabe ist es, eine Dosiermechanik eines Semidisposable Pen oder anderen Verabreichungsgeräts so zu gestalten, dass der Zusammenbau des Geräts bei einem Austausch eines Reservoirmoduls vereinfacht wird.

Ein Verabreichungsgerät, wie die Erfindung es betrifft, weist ein Gehäuse, ein Reservoir für ein ausschüttbares, vorzugsweise injizierbares Produkt und einen Kolben auf, der in dem Reservoir in eine Vorschubrichtung auf einen Reservoirauslass zu verschiebbar aufgenommen ist, so dass durch eine Verschiebung des Kolbens in die Vorschubrichtung Produkt durch den Reservoirauslass hindurch ausgeschüttet wird. Das Reservoir kann von einem Behältnis gebildet werden, das von dem Gehäuse aufgenommen ist. So kann insbesondere eine Ampulle das Reservoir bilden. Das Reservoir kann grundsätzlich aber auch unmittelbar von dem Gehäuse selbst gebildet werden, d.h. ohne Zwischenschaltung eines Produktbehältnisses. Bei dem Produkt handelt es sich vorzugsweise um eine Flüssigkeit für medizinische, therapeutische, diagnostische, pharmazeutische oder kosmetische Anwendungen. So kann das Produkt beispielsweise Insulin, ein Wachstumshormon oder auch flüssige oder breiförmige Nahrung sein. Das Verabreichungsgerät wird vorzugsweise in solchen Verwendungen eingesetzt, in denen ein Benutzer sich das Produkt selbst verabreicht, wie dies beispielsweise in der Diabetestherapie üblich ist. Die Verwendung im stationären und ambulanten Bereich durch Ärzte oder geschultes Personal soll jedoch nicht ausgeschlossen sein.

Das Verabreichungsgerät umfasst ferner eine Kolbenstange, die dazu dient, den Kolben in die Vorschubrichtung zu bewegen. Die Kolbenstange kann mit dem Kolben fest, d.h. ständig, verbunden sein, worunter auch eine einstückige Ausbildung von Kolben und Kolbenstange verstanden werden soll. In bevorzugter Ausführung sind der Kolben und die Kolbenstange jedoch als separate Bauteile ausgeführt, und es drückt zum Zwecke der Produktausschüttung die Kolbenstange mit einem vorderen Ende gegen eine Rückseite des Kolbens.

Des Weiteren umfasst das Verabreichungsgerät ein Dosier- und Antriebselement, mit dem relativ zu dem Gehäuse eine Dosierbewegung zur Auswahl einer Produktdosis und eine Ausschüttbewegung zur Ausschüttung der Produktdosis ausführbar sind. Die Ausschüttbewegung erfolgt vorzugsweise in die Vorschubrichtung, und die Dosierbewegung ist vorzugsweise eine Drehbewegung um eine zu der Vorschubrichtung parallele Achse. Das Dosier- und Antriebselement ist mit der Kolbenstange in einem Eingriff, der eine Mitnahme der Kolbenstange bei der Dosierbewegung bewirkt, aber eine Ausschüttbewegung des Dosier- und Antriebselements relativ zu der Kolbenstange nicht behindert oder zumindest zulässt. Vorzugsweise ist der Eingriff des Dosier- und Antriebselements mit der Kolbenstange formschlüssig. Falls die Dosierbewegung eine Drehbewegung ist, wird durch den Eingriff zwischen dem Dosier- und Antriebselement und der Kolbenstange eine um die Drehachse der Drehbewegung verdrehgesicherte Verbindung geschaffen.

Schließlich umfasst das Verabreichungsgerät ein Dosiseinstellglied, das mit der Kolbenstange und dem Gehäuse je in einem Eingriff steht. Durch den Eingriff mit der Kolbenstange einerseits und den Eingriff mit dem Gehäuse andererseits wird bewirkt, dass das Dosiseinstellglied nur gemeinsam mit der Kolbenstange in die Vorschubrichtung bewegbar ist und durch die Dosierbewegung relativ zu der Kolbenstange entgegen der Vorschubrichtung bewegt wird. Das Dosiseinstellglied wird von dem Dosier- und Antriebselement bei dessen Ausschüttbewegung in die Vorschubrichtung bewegt. Es vollführt dabei gemeinsam mit der Kolbenstange selbst eine Ausschüttbewegung, die auf den Kolben übertragen wird und zur Produktausschüttung führt.

Der Eingriff zwischen dem Dosiseinstellglied und der Kolbenstange ist vorzugsweise ein Gewindeeingriff. In diesem Falle ist die Kolbenstange mit einem Gewinde um die Längsachse der Kolbenstange versehen. Der Eingriff kann jedoch auch anders gebildet werden, beispielsweise in der Art einer Ratsche. Solch ein Zahneingriff wird jedoch bevorzugt eingesetzt, um eine Bewegung der Kolbenstange entgegen der Vorschubrichtung zu verhindern.

Da das Dosier- und Antriebselement bei seiner Ausschüttbewegung nicht unmittelbar auf die Kolbenstange, sondern auf das Dosiseinstellglied wirkt, wie dies grundsätzlich bereits aus der WO 97/17096 bekannt ist, kann ein stets gleichlanger Ausschütthub erzielt werden. Da im Unterschied zu dem bekannten Injektionsgerät jedoch das Dosier- und Antriebselement verdrehgesichert mit der Kolbenstange verbunden und das Dosiseinstellglied mit dem Gehäuse in Eingriff steht, um einen Dosierhub des Dosiseinstellglieds entgegen der Vorschubrichtung zu erhalten, wird das Verabreichungsgerät schlanker. Das Dosier- und Antriebselement muss nämlich nicht mehr das Dosiseinstellglied umgreifen, wie dies bei dem bekannten Gerät der Fall ist, um den Dosierhub der dortigen Dosiseinstellmutter zu bewirken. Es genügt, wenn das Dosier- und Antriebselement im Zuge seiner Ausschüttbewegung ganz einfach mit einer vorderen Stirnfläche gegen das Dosiseinstellglied stößt, um dieses für die Ausschüttung zusammen mit der Kolbenstange vorzuschieben. Dementsprechend können das Dosier- und Antriebselement und das Dosiseinstellglied vorteilhafterweise in Bezug auf die Vorschubrichtung überlappungsfrei hintereinander angeordnet sein. Das Dosier- und Antriebselement kann ein sehr einfach geformtes Teil sein, das in Bezug auf die Dosierung lediglich noch so geformt sein muss, dass die verdrehgesicherte, verschiebliche Verbindung mit der Kolbenstange hergestellt werden kann. Durch den Wegfall des Eingriffs zwischen dem Dosier- und Antriebselement und dem Dosiseinstellglied zum Zwecke der Dosierung wird ein Eingriff zwar zwischen dem Dosiereinstellglied und dem Gehäuse erforderlich, hierdurch wird jedoch quer zu der Kolbenstange kein zusätzlicher Raum benötigt. Das Gehäuse bildet für das Dosiseinstellglied vorzugsweise eine in die Vorschubrichtung weisende Linearführung.

Besonders vorteilhaft ist die Erfindung bei sogenannten Semidisposable Verabreichungsgeräten, insbesondere Semidisposable Injektionspens. Solche Verabreichungsgeräte weisen ein Reservoirmodul auf, das nicht nur das Reservoir für das Produkt umfasst, sondern auch die Kolbenstange hält. Nach einer Entleerung des Reservoirs wird das gesamte Reservoirmodul einschließlich Kolbenstange gegen ein neues Reservoirmodul mit gefülltem Reservoir ausgetauscht. Ein hinterer Teil solch eines Semidisposable Verabreichungsgeräts umfasst das Dosier- und Antriebselement und üblicherweise eine Zähl- und Anzeigeeinrichtung. Dieser im Allgemeinen technisch aufwendige und daher teure Teil des Verabreichungsgeräts ist als wiederverwendbares Teil konzipiert und kann wiederholt mit einem neuen Reservoirmodul verbunden werden. Das Reservoirmodul kann dagegen als Wegwerfteil konzipiert sein, woher die Bezeichnung "Semidisposable" rührt. Bei solchen Geräten wird der Zusammenbau des neuen Reservoirmoduls und des hinteren Geräteteils mit dem Dosier- und Antriebselement durch die Erfindung erleichtert, da bei dem Zusammenbau kein Eingriff zwischen dem Dosier- und Antriebselement des hinteren Geräteteils und dem Dosiseinstellglied, das Bestandteil des Reservoirmoduls ist, hergestellt werden muss.

Die Dosier- und Antriebsvorrichtung kann manuell, halb automatisch oder voll automatisch arbeiten. Im ersten Fall wird sowohl die rotatorische Dosierbewegung als auch die translatorische Ausschüttbewegung manuell ausgeführt. Im zweiten Fall wird entweder die Dosierbewegung oder die Ausschüttbewegung manuell ausgeführt, und die andere Bewegung wird motorisch oder mittels einer anderen Art der Kraftbeaufschlagung, beispielsweise mittels Federkraft bewirkt, wenn der Verwender die entsprechende Bewegung durch einen Betätigungshandgriff ausgelöst hat. Im dritten Fall, der vollautomatischen Dosier- und Antriebsvorrichtung werden die Dosierbewegung und die Ausschüttbewegung motorisch oder mittels einer anderen Kraft, beispielsweise Federkraft, bewirkt. Es wird in diesem Fall manuell lediglich die Dosis ausgewählt, beispielsweise mittels einer oder mehreren Tasten, und es wird die Ausschüttbewegung ebenfalls durch einen eigenen, entsprechenden Betätigungshandgriff des Verwenders ausgelöst. In den meisten Ausführungen ist das erfindungsgemäße Verabreichungsgerät mit einer manuellen Dosier- und Antriebsvorrichtung ausgerüstet, die dann als Dosier-und Betätigungsvorrichtung bezeichnet wird. Soweit daher von einer Dosier- und Betätigungsvorrichtung die Rede ist, wird damit die manuelle Ausführung bezeichnet. Soweit von einer Dosier- und Antriebsvorrichtung die Rede ist, soll hierdurch keine Beschränkung in Bezug auf manuell, halb automatisch oder voll automatisch vorgenommen werden, sondern es soll jede der Ausführungen umfasst sein. Der Begriff "Dosier- und Betätigungsmodul" wird jedoch im Zusammenhang mit sämtlichen Ausführungen der Dosier- und Antriebsvorrichtung verwendet.

Die Dosier- und Antriebsvorrichtung kann ein Dosierelement, das die Dosierbewegung ausführt; und separat ein Antriebselement aufweisen, das die Ausschüttbewegung ausführt. Vorzugsweise werden die Dosierbewegung und Ausschüttbewegung jedoch von dem gleichen Körper der Dosier- und Antriebsvorrichtung ausgeführt, der im Folgenden daher auch als Dosier- und Antriebselement oder als Dosier- und Betätigungselement bezeichnet wird.

Bei dem Produkt handelt es sich vorzugsweise um ein Fluid, besonders bevorzugt eine Flüssigkeit, für eine medizinische, therapeutische, diagnostische, pharmazeutische oder kosmetische Anwendung. Das Produkt kann beispielsweise Insulin, ein Wachstumshormon oder auch dünn- bis dickflüssige, breiförmige Nahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Die Verwendung im stationären und ambulanten Bereich durch geschultes Personal soll jedoch nicht ausgeschlossen sein.

Die Produktverabreichung kann bei einem Injektionsgerät mittels Injektionskanüle oder beispielsweise einer Düse für nadellose Injektionen erfolgen. Die Injektion oder Infusion kann insbesondere subkutan oder venös vorgenommen werden, oder auch intramuskulär. Im Falle der Verabreichung per Inhalation kann die ausgewählte Produktdosis aus dem Reservoir beispielsweise in eine Kammer des Inhalationsgeräts ausgeschüttet und mittels einer Zerstäubungseinrichtung für die Inhalation zerstäubt werden. Denkbar ist ferner eine orale Einnahme oder eine Verabreichung über die Speiseröhre, um nur einige Beispiele der Verabreichung zu nennen.

Besonders bevorzugt ist das Verabreichungsgerät semidisposable. In diesem Fall ist der vordere Gehäuseabschnitt Träger eines Reservoirmoduls, das nach Entleerung des Reservoirs entsorgt oder in einen Kreislauf zurückgeführt wird, und der hintere Gehäuseabschnitt ist Träger eines Dosier- und Betätigungsmoduls, das in Verbindung mit einem neuen Reservoirmodul wiederholt verwendbar ist. Da das Reservoirmodul als Verbrauchsmodul auch separat gehandelt wird, ist es auch separat Gegenstand der Erfindung. Auch das Dosier- und Betätigungsmodul kann separat Gegenstand der Erfindung sein. Ebenso bildet ein System aus einem Verabreichungsgerät und wenigstens einem Reservoirmodul, das das Reservoirmodul des Geräts nach Verbrauch ersetzen kann, einen Gegenstand der Erfindung. Das Konzept des zweigeteilten Verabreichungsgeräts in einen für die nur einmalige Verwendung vorgesehenen Teil und einen für die wiederholte Verwendung vorgesehenen Teil (semidisposable) ist vorteilhaft insbesondere für Injektionspens, aber desweiteren auch für beispielsweise Inhalationsgeräte oder Geräte für die orale Einnahme eines Produkts oder eine künstliche Ernährung.

In den Unteransprüchen werden weitere bevorzugte Ausgestaltungen der Erfindung beschrieben, wobei Merkmale die nur in Bezug auf das Verabreichungsgerät oder nur in Bezug auf ein Reservoirmodul oder ein Dosier-und Betätigungsmodul beansprucht sind, auch bevorzugte Merkmale in Bezug auf den jeweils anderen Anspruchsgegenstand sind.

Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche vorteilhaft weiter. Auch Merkmale, die nur an dem einen Beispiel offenbart sind, bilden das jeweils andere Beispiel weiter oder zeigen eine Alternative auf, soweit nichts Gegenteiliges offenbart wird oder nur der Fall sein kann. Es zeigen:
- Figur 1: zwei Teile eines Reservoirmoduls nach einem ersten Ausführungsbeispiel,
- Figur 2: das aus den zwei Teilen der Figur 1 erhaltene Reservoirmodul,
- Figur 3: ein das Reservoirmodul der Figur 2 umfassendes Injektionsgerät nach dem ersten Ausführungsbeispiel in einem Längsschnitt,
- Figur 4: einen Teil des Injektionsgeräts der Figur 3,
- Figur 5: einen Mechanikhalter des Reservoirmoduls in einem Längsschnitt und zwei Ansichten,
- Figur 6: eine von dem Mechanikhalter gelagerte Blockiereinrichtung für eine Kolbenstange,
- Figur 7: eine Kolbenstange in einem Längsschnitt und einer Stirnansicht,
- Figur 8: eine Verriegelungssperre in einem Längsschnitt, einer Ansicht und einer Draufsicht,
- Figur 9: ein zweites Ausführungsbeispiel eines Injektionsgeräts,
- Figur 10: den Querschnitt A-A der Figur 9,
- Figur 11: den Querschnitt B-B der Figur 9,
- Figur 12: den Querschnitt C-C der Figur 9,
- Figur 13: den Querschnitt D-D der Figur 9,
- Figur 14: den Mechanikhalter des zweiten Ausführungsbeispiels in einer perspektivischen Darstellung,
- Figur 15: den Mechanikhalter der Figur 14 in einer Ansicht,
- Figur 16: den Querschnitt A-A der Figur 15,
- Figur 17: das Dosiseinstellglied des zweiten Ausführungsbeispiels in einer
- Figur 18: perspektivischen Darstellung, das Dosiseinstellglied der Figur 17 in einem Längsschnitt,
- Figur 19: das Dosiseinstellglied der Figur 17 in einer Ansicht,
- Figur 20: das Dosiseinstellglied der Figur 17 in einer Draufsicht,
- Figur 21: einen Teil des Injektionsgeräts nach Figur 3 und
- Figur 22: einen Teil des Injektionsgeräts nach Figur 9.

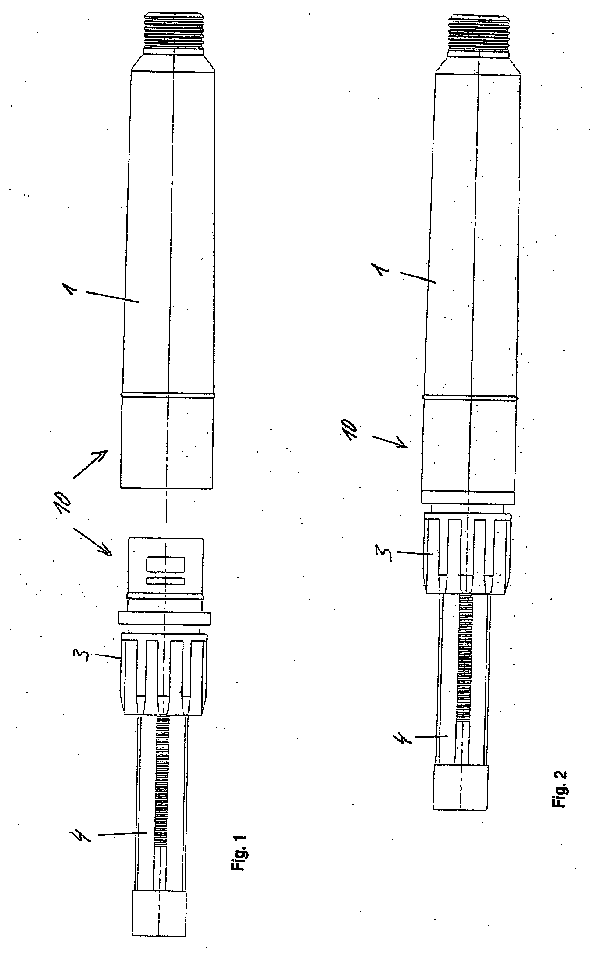

Figur 1 zeigt in einer Ansicht ein Reservoirteil 1 und einen Mechanikhalter 3, die miteinander verbunden werden, um das in Figur 2 dargestellte Reservoirmodul 10 zu bilden.

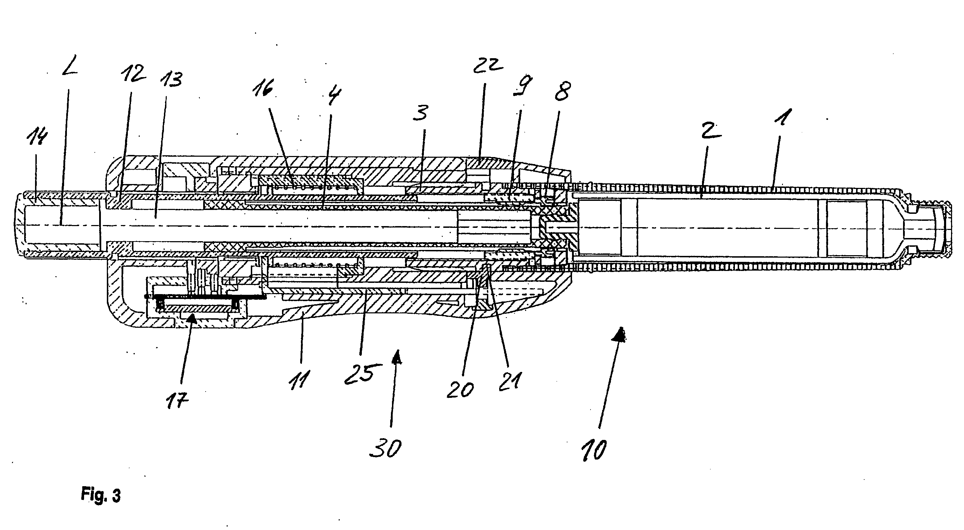

In den Figuren 1 und 2 ist ferner eine Kolbenstange 4 erkennbar, die an einem von dem Reservoirteil 1 abgewandten Ende des Mechanikhalters 3 in den Mechanikhalter 3 hineinragt und von dem Mechanikhalter 3 in eine in Längsachse L der Kolbenstange 4 weisende Vorschubrichtung auf ein von dem Mechanikhalter 3 abgewandtes, vorderes Ende des Reservoirteils 1 zu verschiebbar gelagert wird. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das im Ausführungsbeispiel von einer Ampulle 2 gebildet wird, die in dem Längsschnitt der Figur 3 erkennbar ist. Ein Auslass an dem vorderen Ende der Ampulle 2 wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der hohlen Injektionsnadel und dem Reservoir 2 hergestellt ist.

Figur 3 zeigt das Injektionsgerät in seiner Gesamtheit in einem Längsschnitt. In der Ampulle 2 ist ein Kolben in die Vorschubrichtung auf den am vorderen Ende der Ampulle 2 gebildeten Auslass zu verschiebbar aufgenommen. Durch die Verschiebung des Kolbens in Vorschubrichtung wird Produkt aus der Ampulle 2 verdrängt und durch den Auslass und die Injektionsnadel hindurch ausgeschüttet.

Den Vorschub des Kolbens bewirkt die Kolbenstange 4, die mit ihrem vorderen Ende gegen den Kolben drückt und dadurch bei ihrem eigenen Vorschub den Kolben in die Vorschubrichtung bewegt. Die Kolbenstange 4 wird von dem Mechanikhalter 3 so gehalten, dass sie nach Überwindung eines gewissen Widerstands in die Vorschubrichtung, nicht jedoch entgegen der Vorschubrichtung bewegt werden kann. Die Rückwärtsbewegung der Kolbenstange 4 entgegen der Vorschubrichtung wird von einer Blockiereinrichtung 8 verhindert. Die Blockiereinrichtung 8 wird von dem Mechanikhalter 3 axial fixiert, d.h. sie ist in dem Mechanikhalter 3 so gehalten, dass sie in und gegen die Vorschubrichtung nicht bewegbar ist. Allerdings wird sie von dem Mechanikhalter 3 um die Längsachse L drehbar gelagert. Die Blockiereinrichtung 8 erzeugt auch den Widerstand, der für die Vorwärtsbewegung überwunden werden muss.

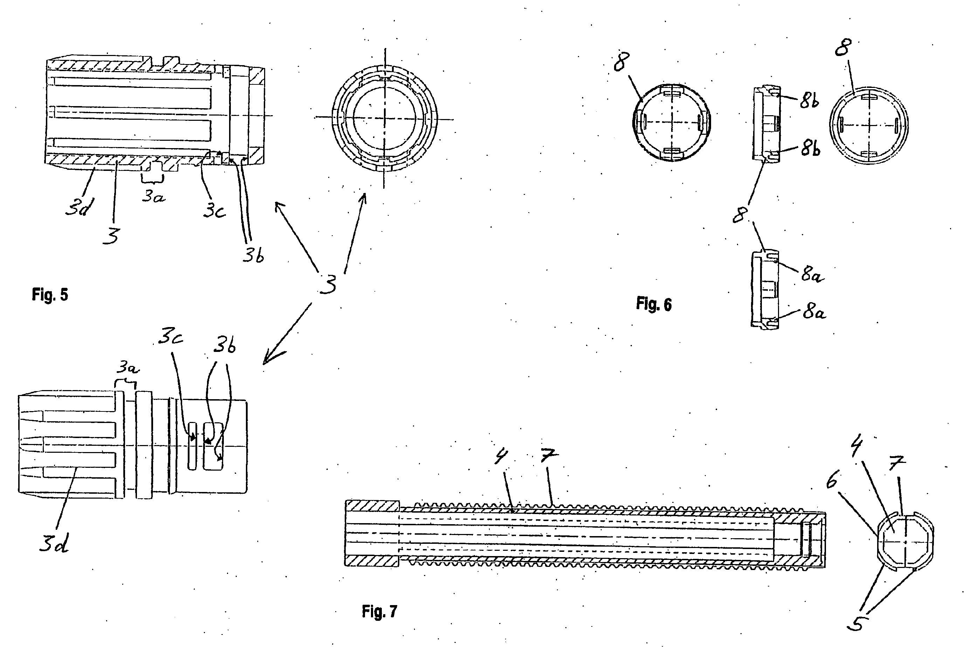

Die Blockiereinrichtung 8 ist in Figur 6 alleine dargestellt. Sie wird von einem einteiligen Ringelement gebildet, das an dem Mechanikhalter 3 zwischen zwei einander zugewandt beabstandeten Schultern 3b, die von einem Innenmantel des Mechanikhaltes 3 radial nach innen vorstehen, um die Längsachse L drehbar anliegt. Die Schultern 3b bilden eine Fixiereinrichtung für die axiale Fixierung der Blockiereinrichtung 8. Die Lagerung der Blockiereinrichtung 8 in dem Mechanikhalter 3 ist am besten aus der Darstellung des Mechanikhalters 3 in Figur 5 ersichtlich.

In dem Mechanikhalter 3 ist ferner ein Dosiseinstellglied 9 aufgenommen. Das Dosiseinstellglied 9 ist als Gewindemutter ausgebildet und steht mit einem Außengewinde der Kolbenstange 4 in einem Gewindeeingriff. Das Dosiseinstellglied 9 wird von dem Mechanikhalter 3 verdrehgesichert, aber in und gegen die Vorschubrichtung axial linear bewegbar geführt. Die Kolbenstange 4 und das Dosiseinstellglied 9 bilden einen Spindeltrieb, um die zu verabreichende Produktdosis auszuwählen.

Der Ampullenhalter 1 und der Mechanikhalter 3 sind verdreh- und verschiebegesichert miteinander verbunden und bilden zusammen das Reservoirmodul 10 des Injektionsgeräts, wobei dieses Reservoirmodul 10 die von dem Mechanikhalter 3 mittels der Blockiereinrichtung 8 gehaltene Kolbenstange 4 und das Dosiseinstellglied 9 umfasst. Der Ampullenhalter 1 und der Mechanikhalter 3 bilden zusammen einen vorderen Gehäuseabschnitt des Injektionsgeräts. Mit diesem vorderen Gehäuseabschnitt 1, 3 ist ein hinterer Gehäuseabschnitt 11 formschlüssig verbunden. Der hintere Gehäuseabschnitt 11 bildet den Träger eines Dosier- und Betätigungselements 12 und zusammen mit demselben sowie Teilen einer Verriegelungseinrichtung und weiteren Teilen ein Dosier- und Betätigungsmodul 30 des Injektionsgeräts.

Eine Dosier- und Betätigungsvorrichtung umfasst mit Ausnahme des Dosiseinstellglieds 9, der Kolbenstange 4 und der Blockiereinrichtung 8 die anderen Komponenten für die Auswahl der Produktdosis und die Betätigung des Injektionsgeräts. Insbesondere umfasst sie das Dosier- und Betätigungselement 12. Ferner umfasst die Dosier- und Betätigungsvorrichtung eine Zähl- und Anzeigeeinrichtung 17 zum Zählen und optischen Anzeigen der ausgewählten Produktdosis. Nicht zuletzt die Zähl- und Anzeigeeinrichtung 17 macht das Dosier-und Betätigungsmodul 30 zu einem hochwertigen und daher hochpreisigen Teil des Injektionsgeräts. Während das vergleichsweise preiswerte Reservoirmodul 10 als Einwegmodul konzipiert ist, ist das Dosier- und Betätigungsmodul 30 für die mehrmalige Verwendung mit immer wieder neuen Reservoirmodulen 10 bestimmt.

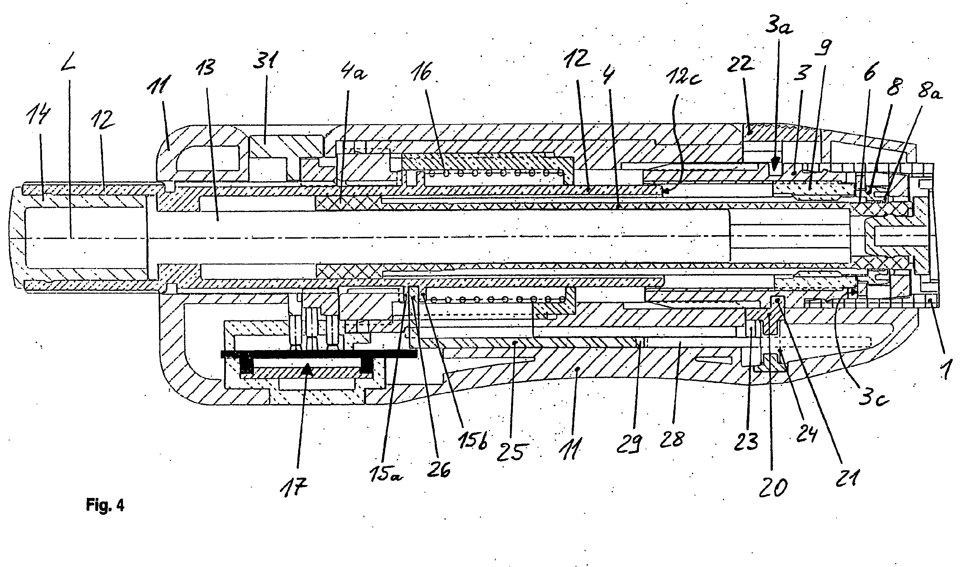

Für die Auswahl der Produktdosis, d.h. für die Dosierung, wird das Dosier- und Betätigungselement 12 um die Längsachse L drehbar und ferner in und gegen die Vorschubrichtung entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ein hinterer Abschnitt des Dosier- und Betätigungselements 12 ragt über ein hinteres Ende des Gehäuseabschnitts 11 hinaus. In das Dosier- und Betätigungselement 12 ist von hinten ein stangenförmiger Dosiermitnehmer 13 bis gegen eine radial nach innen vorragende Schulter des Dosier- und Betätigungselements 12 eingeschoben. Ferner ist an dem hinteren Ende ein Verschluss 14 bis gegen den Dosiermitnehmer 13 in das Dosier- und Betätigungselement 12 eingeschoben. Der Dosiermitnehmer 13 wird zwischen der radial vorragenden Schulter des Dosier-und Betätigungselements 12 und dem Verschluss 14 relativ zu dem Dosier- und Betätigungselement 12 axial fixiert. Der Dosiermitnehmer 13 ist mit dem Dosier-und Betätigungselement 12 außerdem verdrehgesichert verbunden. Der Dosiermitnehmer 13 ragt zum Zwecke der Dosierung von hinten in die hohle Kolbenstange 4 hinein. Die Kolbenstange 4 weist einen Verbindungabschnitt 4a auf (Fig. 4), der mit dem Dosiermitnehmer 13 in solch einem Eingriff ist, dass die Kolbenstande 4 und der Dosiermitnehmer 13 und mit diesem auch das Dosier-und Betätigungselement 12 relativ zueinander um die gemeinsame Längsachse L nicht verdrehbar, aber entlang der Längsachse L in und gegen die Vorschubrichtung relativ zueinander bewegbar sind. Hierfür ist der Verbindungsabschnitt 4a als Linearführung für den Dosiermitnehmer 13 ausgebildet.

Eine Rückstelleinrichtung 16 spannt das Dosier- und Betätigungselement 12 elastisch gegen die Vorschubrichtung in die in den Figuren 3 und 4 dargestellte Ausgangsstellung. In der Ausgangsstellung kann durch Drehung des Dosier- und Betätigungselements 12 um die Längsachse L die Dosierung vorgenommen werden. Anschließend kann aus der Ausgangsstellung heraus durch Axialverschiebung des Dosier- und Betätigungselements 12 die Ausschüttung der ausgewählten Produktdosis bewirkt werden. Die Rückstelleinrichtung 16 wird von einer als Druckfeder wirkenden Spiralfeder gebildet, die in einem Ringspalt um das Dosier- und Betätigungselement 12 aufgenommen ist und zwischen einer radial nach innen ragenden Schulter des Gehäuseabschnitts 11 und einer gegenüberliegend zugewandt radial nach außen ragenden Schulter des Dosier-und Betätigungselements 12 axial abgestützt ist.

Die Blockiereinrichtung 8 erfüllt eine Doppelfunktion. Zum einen stellt sie mit ihren Sperrelementen 8a sicher, dass die Kolbenstange 4 relativ zu dem Mechanikhalter 3 und damit insbesondere relativ zu dem in der Ampulle 2 aufgenommenen Kolben nicht entgegen der Vorschubrichtung zurückbewegt werden kann. In ihrer Doppelfunktion verhindert die Blockiereinrichtung 8 ferner als Bremse eine Vorwärtsbewegung der Kolbenstange 4 bei dem Dosiervorgang, bei dem das Dosiseinstellglied 9 entgegen der Vorschubrichtung axial auf das Dosier- und Betätigungselement 12 zu bewegt wird.

In der in den Figuren 3 und 4 dargestellten Ausgangsstellung vor einer Dosierung liegt das Dosiseinstellglied 9 in Vorschubrichtung auf Anschlag gegen einen von dem Mechanikhalter 3 gebildeten Ausschüttanschlag 3c (Figur 5). Die Kolbenstange 4 steht in einem permanenten Berührungskontakt mit dem Kolben. Zum Zwecke der Dosierung wird das Dosiseinstellglied 9 durch den Gewindeeingriff mit der Kolbenstange 4 und die Linearführung durch den Mechanikhalter 3 von dem Ausschüttanschlag 3c weg auf das Dosier- und Betätigungselement 12 zu bewegt. Hierdurch wird ein lichter Abstand zwischen einer rückwärtigen Anschlagfläche des Dosiseinstellglieds 9 und einer vorderen Anschlagfläche des Dosier- und Betätigungselements 12 verringert, andererseits jedoch der lichte Abstand zwischen einer vorderen Anschlagfläche des Dosiseinstellglieds 9 und dem Ausschüttanschlag 3c vergrößert. Der letztgenannte Abstand zwischen dem Ausschüttanschlag 3c und dem Dosiseinstellglied 9 ist die Weglänge, um die im Zuge der Ausschüttbewegung des Dosier- und Betätigungselements 12 das Dosiseinstellglied 9 und wegen des Gewindeeingriffs auch die Kolbenstange 4 in die Vorschubrichtung bewegt werden. Der Ausschüttanschlag 3c bildet einen vorderen Translationsanschlag. Bei der Ausschüttbewegung drückt die Kolbenstange 4 mit ihrem vorderen Ende, das von einem mit der Kolbenstange 4 in und gegen die Vorschubrichtung nicht bewegbar verbundenen Stempelkörper gebildet wird, gegen den Kolben und drückt den Kolben in die Vorschubrichtung auf den Auslass der Ampulle 2 zu vor. Die Längsachse L bildet die Rotations- und Translationsachse der Bewegungen, die zum Zwecke der Dosierung und Produktausschüttung ausgeführt werden.

Der Abstand, den das Dosiseinstellglied 9 und das Dosier- und Betätigungselement 12 vor dem Dosiervorgang zwischen sich aufweisen, wenn das Dosiseinstellglied 9 auf Anschlag gegen den Ausschüttanschlag 3c liegt, entspricht der maximalen Produktdosis, die ausgewählt und im Zuge einer Ausschüttung ausgeschüttet werden kann. Die Hubbewegung des Dosier- und Betätigungselements 12 ist bei jeder Ausschüttung gleich lang. Es wird durch die Dosierung lediglich der Abstand des Dosiseinstellglieds 9 von dem Ausschüttanschlag 3c und damit die im Zuge der Ausschüttung von dem Dosier-und Betätigungselement 12 und dem Dosiseinstellglied 9 gemeinsam zurücklegbare Weglänge eingestellt.

Die Bremsfunktion der Blockiereinrichtung 8 und der hierfür zwischen der Kolbenstange 4 und der Blockiereinrichtung 8 bestehende Bremseingriff werden aus der Zusammenschau der Figuren 6 und 7 deutlich. Zum einen weist die Blockiereinrichtung 8 für den Bremseingriff zwei Bremselemente 8b auf, die, wie bereits die Sperrelemente 8a, je von einem elastisch nachgebenden Schnapper gebildet werden. Die Blockiereinrichtung 8 wird im Ausführungsbeispiel von einem einzigen Ringelement gebildet, von dem an einer Stirnseite vier elastische Schnapper axial abragen. Die Schnapper sind gleichmäßig über den Umfang des Ringelements verteilt angeordnet. Zwei einander gegenüberliegende Schnapper bilden die Sperrelemente 8a und die zwei weiteren, einander ebenfalls gegenüberliegend angeordneten Schnapper bilden die Bremselemente 8b.

Die Kolbenstange 4 weist dementsprechend zwei am Außenmantel an gegenüberliegenden Seiten ausgebildete und in Längsrichtung der Kolbenstange 4 sich erstreckende Rückzugsperreinrichtungen 6 und zwei an ebenfalls einander gegenüberliegenden Seiten in Längsrichtung der Kolbenstange 4 sich erstreckende Vorschubbremseinrichtungen 7 auf. Das Gewinde der Kolbenstange 4 für den Gewindeeingriff mit dem Dosiseinstellglied 9 wird von vier verbleibenden Gewindeabschnitten 5 gebildet, die sich über nahezu die gesamte Länge der Kolbenstange 4 erstrecken. Die Rückzugsperreinrichtungen 6 und die Vorschubbremseinrichtungen 7 werden je von einer Zahnreihe gebildet. Während die Zähne der Rückzugsperreinrichtungen 6 jedoch als in Vorschubrichtung gepfeilte Sägezähne mit nach hinten weisenden, quer zur Vorschubrichtung sich erstreckenden Sperrflächen gebildet sind, weisen die beiden Zahnreihen, die die Vorschubbremseinrichtungen 7 bilden, nach vorne weisende Sperrflächen mit einer vergleichbaren Sperrwirkung nicht auf. Die Zähne der Vorschubbremseinrichtungen 7 weisen je ein im Vergleich zu den Rückzugssperreinrichtungen 6 weicheres Zahnprofil auf. Der Bremseingriff der Blockiereinrichtung 8 mit den Vorschubbremseinrichtungen 7 der Kolbenstange 4 soll eine Vorschubbewegung der Kolbenstange 4 nämlich nicht verhindern, sondern nur erschweren, um sicherzustellen, dass die Kolbenstange 4 nicht bei der Dosierung in Vorschubrichtung bewegt wird. Die Zähne der Vorschubbremseinrichtungen 7 sind an ihren Vorderseiten und die Bremselemente 8b sind an ihren Rückseiten, die die Vorderseiten der Zähne der Vorschubbremseinrichtung 7 berühren, so geformt, dass zur Überwindung des Bremseingriffs eine Schwellkraft überwunden werden muss, die bei der Dosierung nicht erreicht wird. Diese Schwellkraft ist größer als die Kraft, die erforderlich ist, um die Zähne der Rückzugssperreinrichtungen 6 über die Sperrelemente 8a in Vorschubrichtung zu bewegen. Vorzugsweise ist die Schwellkraft wenigstens doppelt so groß wie die anfängliche Reibkraft zwischen den Rückzugssperreinrichtungen 6 und den Sperrelementen 8a. Die Reibkraft zwischen letzteren vergrößert sich auch erst im Verlaufe der Vorschubbewegung allmählich zwischen zwei aufeinanderfolgenden Sperreingriffen. Die Schwellkraft des Bremseingriffs muss hingegen in jedem Sperreingriff unmittelbar am Beginn der Vorschubbewegung von einem Sperreingriff in den nächst folgenden aufgebracht werden. Allerdings soll die Schwellkraft nicht so groß sein, dass sie vom Verwender bei der Ausschüttung als störend empfunden wird.

Eine unerwünschte Vorschubbewegung der Kolbenstange als Reaktion auf die Bewegung des Dosiseinstellglieds 9 bei der Dosisauswahl kann grundsätzlich auch allein durch den Sperreingriff der Blockiereinrichtung 8 bewirkt werden. Allerdings wird solch eine Bewegung wegen des Bremseingriffs sicherer verhindert als durch den Sperreingriff allein.

Die Verbindung zwischen dem Reservoirmodul 10 und dem Dosier- und Betätigungsmodul 30 ist formschlüssig. Zum einen besteht zwischen dem Mechanikhalter 3 und dem Gehäuseabschnitt 11 ein Verriegelungseingriff, der eine Relativbewegung in axialer Richtung verhindert. Über den Verriegelungseingriff hinaus sind der vordere Gehäuseabschnitt 1, 3 und der hintere Gehäuseabschnitt 11 unmittelbar aneinander axial linear geführt, um eine Relativdrehung bei dem Verbinden und im verbundenem Zustand zu verhindern. In Figur 5 sind die Axialführungen 3d des Mechanikhalters 3, die mit einem oder mehreren entsprechenden Eingriffselementen des hinteren Gehäuseabschnitts 11 die Linearführung bilden, gut zu erkennen. Die Axialführungen 3d werden von Führungsflächen an Führungsrippen gebildet; sie könnten auch von Führungsflächen in axial sich erstreckenden Vertiefungen gebildet werden. Auf diese Weise werden axiale Führungskanäle erhalten. Die Führungsrippen sind axial verjüngt, so dass in die Führungskanäle führende Einführtrichter für das eine oder die mehreren Eingriffselemente des hinteren Gehäuseabschnitts 11 gebildet werden. Zur noch besseren Zentrierung der Gehäuseabschnitte 1, 3 und 11 zu Beginn des Verbindens sind die Führungsrippen auch noch in radialer Richtung verjüngt. Das eine oder die mehreren Eingriffselemente des hinteren Gehäuseabschnitts 11 ist oder sind vorzugsweise wie die Axialabschnitte 3d an der Mantelgegenfläche, d.h. der Innenmantelfläche, des hinteren Gehäuseabschnitts 11 gebildet.

Der Verriegelungseingriff besteht zwischen einem weiblichen, ersten Verriegelungselement 3a des Mechanikhalters 3 (Figur 5) und einem Verriegelungsring 20, der mit dem hinteren Gehäuseabschnitt 11 radial bewegbar, aber axial nicht bewegbar verbunden ist. Der Verriegelungsring 20 bildet ein unmittelbar in das erste Verriegelungselement 3a radial eingreifendes männliches, zweites Verriegelungselement 21. Zwischen dem ersten Verriegelungselement 3a und dem zweiten Verriegelungselement 21 besteht eine Schloss/Riegel-Verbindung, die axiale Relativbewegungen zwischen dem Reservoirmodul 10 und dem Dosier- und Betätigungsmodul 30 verhindert.

Die Figuren 3 und 4 zeigen das Verriegelungselement 21 im Verriegelungseingriff mit dem Verriegelungselement 3a. Das Verriegelungselement 3a wird von einem Ringsteg und einer Nut gebildet, die an dem Außenmantel des Mechanikhalters 3 umlaufen. Der Ringsteg bildet eine hintere Seitenwand der Nut. Das zweite Verriegelungselement 21 wird von einem Nocken gebildet, der von dem Innenmantel des Verriegelungsrings 20 radial einwärts vorragt und im Verriegelungseingriff von einer Rückstelleinrichtung 24 radial einwärts über eine Innenmantelfläche des hinteren Gehäuseabschnitts 11 vorstehend in das aufnehmende Verriegelungselement 3a hineingedrückt wird. Der Verriegelungsring 20 ist in seiner Gesamtheit in Radialrichtung mittels der Rückstelleinrichtung 24 an einer von dem Gehäuseabschnitt 11 gebildeten Innenmantelfläche abgestützt, so dass die Rückstelleinrichtung 24 etwa in radialer Verlängerung des Verriegelungselements 21 gegen den Außenmantel des Verriegelungsrings 20 drückt. Der Verriegelungsring 20 umgibt den Mechanikhalter 3 und ist in seiner Gesamtheit gegen die rückstellende Kraft der Rückstelleinrichtung 24 radial hin und her bewegbar, so dass das zweite Verriegelungselement 21 in und aus dem Verriegelungseingriff mit dem ersten Verriegelungselement 3a bringbar ist. Der hintere Gehäuseabschnitt 11 bildet eine enge Gleitführung für die Radialbewegung des Verriegelungsrings 20. An seiner dem Verriegelungselement 21 radial gegenüberliegenden Seite bildet der Verriegelungsring 20 einen Entriegelungsknopf 22 für den Verwender. Zur radialen Führung der als Druckfeder ausgebildeten Rückstelleinrichtung 24 ragt von der dem Verriegelungselement 21 abgewandten Außenmantelfläche des Verriegelungsrings 20 ein Führungsnocken radial ab.

In Umfangsrichtung zu beiden Seiten dieses Führungsnockens und axial hinter dem Führungsnocken ragen von der Außenmantelfläche des Verriegelungsrings 20 ferner zwei Sperrnocken 23 ab, die in Radialrichtung nach außen gegen eine Verriegelungssperre 25 drücken. Aufgrund der Anlage der Sperrnocken 23 gegen die Verriegelungssperre 25 wird eine Radialbewegung des Verriegelungselements 21 verhindert, die zu einem Lösen des Verriegelungseingriffs führen könnte. Der zwischen den Verriegelungselementen 3a und 21 bestehende Verriegelungseingriff wird somit durch die Verriegelungssperre 25 gesichert. Diese Sicherung besteht in jeder Verschiebeposition des Dosier- und Betätigungselements 12 mit Ausnahme einer Freigabestellung, die das Dosier-und Betätigungselement 12 am Ende seiner Ausschüttbewegung einnimmt. Die Freigabestellung fällt daher mit der vordersten Verschiebeposition zusammen, die das Dosier- und Betätigungselement 12 dann einnimmt, wenn es im Zuge seiner Ausschüttbewegung an das Dosiseinstellglied 9 und das Dosiseinstellglied 9 seinerseits gegen den Ausschüttanschlag 3c des Mechanikhalters 3 anstößt. Solange das Dosier- und Betätigungsmodul 30 mit dem Reservoirmodul noch nicht verbunden ist, wird ein mechanischer Anschlag für das Dosier- und Betätigungselement 12 von einem Anschlagelement 31 der Dosier- und Betätigungsvorrichtung gebildet. Im Ausführungsbeispiel bildet ein Resethalterring, der einem Reset der Anzeige 17 dient, das Anschlagelement 31. Der Anschlag des Dosier- und Betätigungselement 12 gegen dieses Anschlagelement 31 definiert die Freigabestellung des Dosier- und Betätigungselements 12 in diesem Falle, wobei die durch das Anschlagelement 31 definierte Freigabestellung derjenigen entspricht, die durch das Anstoßen des Dosiseinstellglieds 9 an dem Ausschüttanschlag 3c definiert wird.

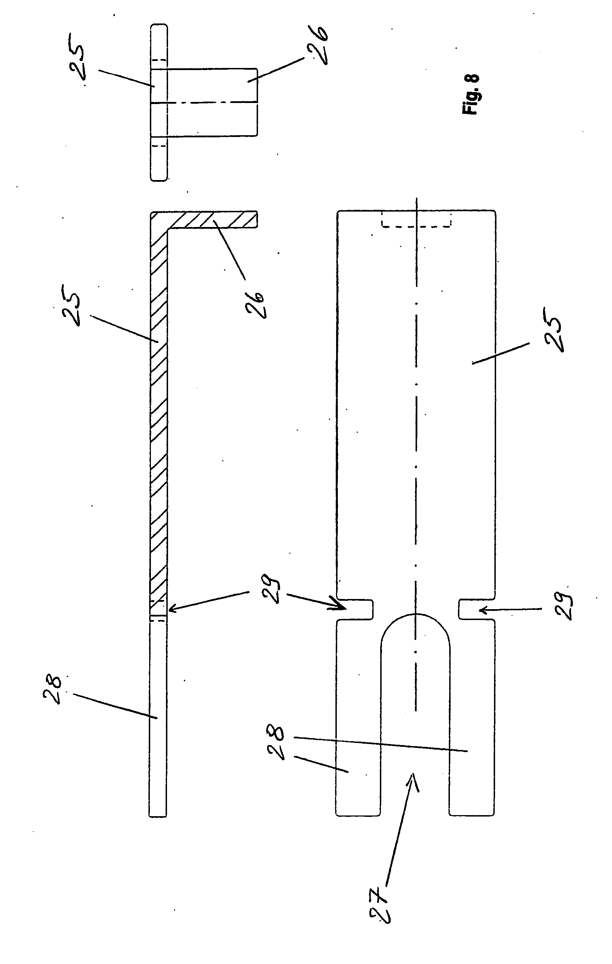

Figur 8 zeigt die Verriegelungssperre 25. Sie wird im Ausführungsbeispiel einstückig von einem Sperrschieber gebildet. Die Verriegelungssperre 25 weist einen plattenförmigen Hauptkörper auf, der sich im montierten Zustand, wie beispielsweise in Figur 4 dargestellt, axial erstreckt. An einem Ende ragt von dem Hauptkörper ein Steg 26 rechtwinklig ab. Im montierten Zustand erstreckt sich der Steg 26 radial bis gegen das Dosier- und Betätigungselement 12. Der Steg 26 dient der Befestigung der Verriegelungssperre 25 an dem Dosier- und Betätigungselement 12, das hierfür zwei axial beabstandet an einer Außenmantelfläche gebildete Ringstege aufweist, die Mitnehmer 15a und 15b bilden. Der vordere Mitnehmer 15b bildet gleichzeitig die Stützschulter für die Rückstelleinrichtung 16. In den zwischen den Mitnehmern 15a und 15b gebildeten Ringraum ragt die Verriegelungssperre 25 mit ihrem Steg 26 ein und wird von den beiden Mitnehmern 15a und 15b axial beidseits eng eingefasst.

An einem von dem Steg 26 abgewandten vorderen Ende ist der Hauptkörper der Verriegelungssperre 25 mit einer Axialausnehmung 27 versehen, die zu dem vorderen Ende der Verriegelungssperre 25 hin offen ist. Auf diese Weise werden beidseits der Ausnehmung 27 axial sich erstreckende Sperrzungen 28 gebildet. Die beiden Sperrnocken 23 des Verriegelungsrings 20 sind so angeordnet, dass je einer dieser Sperrnocken 23 gegen eine der Sperrzungen 28 drückt, solange das Dosier- und Betätigungselement 12 nicht die Freigabestellung einnimmt. Durch die axiale Ausnehmung 27 hindurch erstreckt sich bei der Axialbewegung der Verriegelungssperre 25 die Rückstelleinrichtung 24 für das Verriegelungselement 21.

In dem Hauptkörper der Verriegelungssperre 25 sind ferner Einrückausnehmungen 29 gebildet, die die Freigabestellung des Dosier- und Betätigungselements 12 definieren. Pro Sperrnocken 23 ist je eine Einrückausnehmung 29 vorgesehen. Die Position der Einrückausnehmungen 29 ist so gewählt, dass sie mit den Sperrnocken 23 erst dann in Überdeckung gelangen und dadurch ein Einfahren der Sperrnocken 23 gestatten, wenn das Dosier- und Betätigungselement 12 bis in seine Freigabestellung vorbewegt worden ist.

Es ist klar, dass bei der im Ausführungsbeispiel speziell gewählten Anordnung auch ein einziger Sperrnocken 23 vorgesehen sein könnte und die Verriegelungssperre 25 dementsprechend auch nur eine einzige Einrückausnehmung 29 und gegebenenfalls auch nur eine Sperrzunge 28 aufweisen könnte. Grundsätzlich könnte die Verriegelungssperre ferner einstückig mit dem Dosier- und Betätigungselement 12 gefertigt sein. Die Ausbildung als separates Teil bietet jedoch Vorteile hinsichtlich der Fertigung, der Montage und dem Zusammenwirken des Dosier- und Betätigungselements 12 mit der Kolbenstange 4. In Bezug auf die Einbaulage der Verriegelungssperre 25 ist noch darauf hinzuweisen, dass die Verriegelungssperre 25 an ihrer von dem Verriegelungselement 21 abgewandten Außenseite an einer Innenmantelfläche des Gehäuses 11 abgestützt ist. Auf diese Weise wird die Stabilität der Sicherung für den Verriegelungseingriff erhöht. Vorzugsweise bildet das Gehäuse 11 eine Axialführung für die Verriegelungssperre 25.

Im Folgenden wird die Funktionsweise des Injektionsgeräts beschrieben, wobei angenommen sei, dass ein neues Reservoirmodul 10 und ein bereits wenigstens einmal verwendetes Dosier- und Betätigungsmodul 30 zusammengebaut und anschließend eine erste Produktausschüttung vorgenommen werden.

Das Dosier- und Betätigungsmodul 30 und das neue Reservoirmodul 10 werden axial zueinander ausgerichtet, so dass ihre beiden Längsachsen miteinander fluchten. Anschließend wird das Reservoirmodul 10 mit seinem rückwärtigen Ende in das nach vorne offene Gehäuse 11 des Dosier- und Betätigungsmoduls 30 eingeführt.

Hierbei zentrieren sich der Gehäuseabschnitt 1, 3 und der Gehäuseabschnitt 11 an den verjüngten Enden der Führungsrippen 3d des Mechanikhalters 3. Während des Aufschiebens werden die beiden Gehäuseabschnitte in einer durch die Linearführung vorgegebenen Drehwinkelposition axial aneinander lineargeführt, bis die Gehäuseabschnitte 1, 3 und 11 eine Verbindungsendposition einnehmen, in der der Verriegelungseingriff der Verriegelungselemente 3a und 21 hergestellt werden kann oder von selbst sich einstellt.

Das Dosier- und Betätigungselement 12 ist in vorgegebenen Drehwinkelpositionen relativ zu dem hinteren Gehäuseabschnitt 11 gerastet. Die Linearführung der Gehäuseabschnitte 1, 3 und 11 und die Drehwinkelrastposition des Dosier- und Betätigungselements 12 sind so aufeinander abgestimmt, dass der verdrehgesicherte Eingriff des Dosier- und Betätigungselements 12 mit der Kolbenstange 4 in jeder Rastposition des Dosier- und Betätigungselements 12 und jeder Drehwinkelposition, in der die Gehäuseabschnitte 1, 3 und 11 aneinander lineargeführt sind, hergestellt wird.

Falls das Dosier- und Betätigungselement 12 sich in einer Axialposition relativ zu dem Gehäuseabschnitt 11 befindet, die hinter der Freigabestellung liegt, wird das Verriegelungselement 21 von der Verriegelungssperre 25 in seiner radial innersten Stellung gehalten. In dieser Stellung des Verriegelungselements 21 können das Dosier- und Betätigungsmodul 30 und das Reservoirmodul 10 nicht bis in die Verbindungsendstellung aufeinandergeschoben und deshalb auch nicht miteinander verbunden werden, da der am Außenmantel des Mechanikhalters 3 gebildete Ringsteg, der das erste Verriegelungselement 3a mitbildet, vorher gegen das zweite Verriegelungselement 21 auf Anschlag zu liegen kommt.

Der Ringsteg kann zu einem in tangentialer Richtung kurzen Radialvorsprung reduziert werden, wenn dafür gesorgt ist, dass die Gehäuseabschnitte 1, 3 und 11 nur in der Drehwinkellage zusammengebaut werden können, in der solch ein Vorsprung und das zweite Verriegelungselement 21 in einer axialen Flucht zu liegen kommen. Der Ringsteg oder Radialvorsprung könnte das erste Verriegelungselement 3a auch alleine bilden, da es die wesentliche Funktion des ersten Verriegelungselements 3a ist, die Herstellung der Verbindung zwischen dem Reservoirmodul 10 und dem Dosier- und Betätigungsmodul 30 nur zuzulassen, wenn das Dosier- und Betätigungselement 12 seine Freigabestellung einnimmt. Ist diese Bedingung erfüllt, so würde das Dosier- und Betätigungselement 12 bei der Herstellung der Verbindung zwischen dem Reservoirmodul 10 und dem Dosier- und Betätigungsmodul 30 sicherstellen, dass das Dosiseinstellglied 9 sich in seiner Dosiernullstellung befindet, in der es an dem Ausschüttanschlag 3c des Mechanikhalters 3 anstößt.

Um den die vorstehend erläuterte Bedingung erfüllenden Zustand herzustellen, drückt der Verwender das Dosier- und Betätigungselement 12 axial relativ zu dem hinteren Gehäuseabschnitt 11 bis in die Freigabestellung vor. In dieser Relativstellung zwischen Gehäuseabschnitt 11 und Dosier- und Betätigungselement 12 können die Sperrnocken 23 in die Einrückaufnahmen 29 der Verriegelungssperre 25 bewegt werden. Der Verwender drückt daher nicht nur das Dosier- und Betätigungselement 12 bis wenigstens in die Freigabestellung vor, sondern gleichzeitig auch mittels des Entriegelungsknopfs 22 das erste Verriegelungselement 20 aus dem Verriegelungseingriff. Das Reservoirmodul 10 kann nun axial über den Ringsteg des ersten Verriegelungselements 3a bewegt und weiter in den hinteren Gehäuseabschnitt 11 eingeführt werden. Der Verwender kann den Entriegelungsknopf 22 loslassen. Sobald das erste Verriegelungselement 21 mit dem zweiten Verriegelungselement 3a in Überdeckung gelangt, schnappt es aufgrund der Kraft der Rückstelleinrichtung 24 in das aufnehmende Verriegelungselement 3a ein, so dass der Verriegelungseingriff hergestellt ist. Das Reservoirmodul 10 und das Dosier- und Betätigungsmodul 30 sind nun in Bezug auf die Stellung des Dosiseinstellglieds 9 und der Kolbenstange 4 in definierter Weise miteinander verbunden. Falls das Dosiseinstellglied 9 vor Herstellung des Verriegelungseingriffs noch einen lichten Abstand von dem Ausschüttanschlag 3c aufgewiesen hat, ist dieser Abstand aufgrund der zum Herstellen der Verbindung erforderlichen Einwirkung des Dosier- und Betätigungselements 12 beseitigt. Eine damit einhergehende Produktausschüttung kann hingenommen werden und kann zum Zwecke des Primens der Injektionsnadel sogar erwünscht sein. Die Zähl- und Anzeigeeinrichtung 17 wird vorzugsweise dabei genullt.

In dem derart herbeigeführten, definierten Ausgangszustand kann der Verwender die Dosierung vornehmen. Die Dosierung erfolgt durch Drehung des Dosier- und Betätigungselements 12 um die Längsachse L und relativ zu dem Gehäuseabschnitt 11. Da der Dosiermitnehmer 13 mit dem Dosier- und Betätigungselement 12 verdrehgesichert verbunden ist und seinerseits verdrehgesichert in die Kolbenstange 4 eingreift, nimmt das Dosier- und Betätigungselement 12 bei der rotatorischen Dosierbewegung die Kolbenstange 4 mit. Aufgrund des Gewindeeingriffs zwischen der Kolbenstange 4 und dem Dosiseinstellglied 9 und der Linearführung des Dosiseinstellglieds 9 durch den Mechanikhalter 3 führt das Dosiseinstellglied 9 eine von der Gewindesteigung des gegenseitigen Gewindeeingriffs vorgegebene, axiale translatorische Dosierbewegung in Richtung auf das Dosier- und Betätigungselement 12 aus. Das Dosier- und Betätigungselement 12 bildet einen hinteren Translationsanschlag 12c, der die translatorische Dosierbewegung des Dosiseinstellglieds 9 begrenzt und dadurch den maximal einstellbaren Ausschütthub definiert.

Die Zähl- und Anzeigeeinrichtung 17 zählt die der Drehwinkelstellung des Dosier-und Betätigungselements 12 entsprechenden Dosiseinheiten und zeigt sie optisch an.

Nach Auswahl der gewünschten Produktdosis ist der Dosiervorgang beendet. Die Ausschüttung der gewählten Produktdosis wird mittels der in Vorschubrichtung des Kolbens weisenden Ausschüttbewegung des Dosier- und Betätigungselements 12 bewirkt. Im Zuge seiner Ausschüttbewegung stößt das Dosier- und Betätigungselement 12 gegen das Dosiseinstellglied 9 und nimmt es mit. Indem das Dosiseinstellglied 9 im Zuge der Ausschüttbewegung gegen den Ausschüttanschlag 3c des Mechanikhalters 3 stößt, werden die Ausschüttbewegungen des Dosier- und Betätigungselements 12 und die Produktausschüttung beendet. Nach dem Loslassen des Dosier- und Betätigungselements 12 vorzugsweise wird dieses von dem Rückstelleinrichtung 16 entgegen der Vorschubrichtung wieder in eine neue Ausgangsstellung für eine erneute Dosierung und Produktausschüttung bewegt. Die Zähl- und Anzeigeeinrichtung 17 ist mit dem Dosier- und Betätigungselement 12 vorzugsweise so gekoppelt, dass sie mittlerweile wieder auf Null zurückgestellt worden ist. Gegebenenfalls verfügt sie über Mittel zum Zählen und Anzeigen der bereits insgesamt ausgeschütteten Produktmenge und somit der in der Ampulle 2 verbliebenen Produktrestmenge.

Um das Reservoirmodul 10 von dem Dosier- und Betätigungsmodul 30 zu lösen, wird das Dosier- und Betätigungselement 12 bis in die Freigabestellung, d.h. bis auf Anschlag gegen das Dosiseinstellglied 9, vorbewegt. In dieser Stellung kann der Verwender durch Druck auf den Entriegelungsknopf 22 den Verriegelungseingriff wieder lösen und das Reservoirmodul 10 von dem Dosier-und Betätigungsmodul 30 trennen.

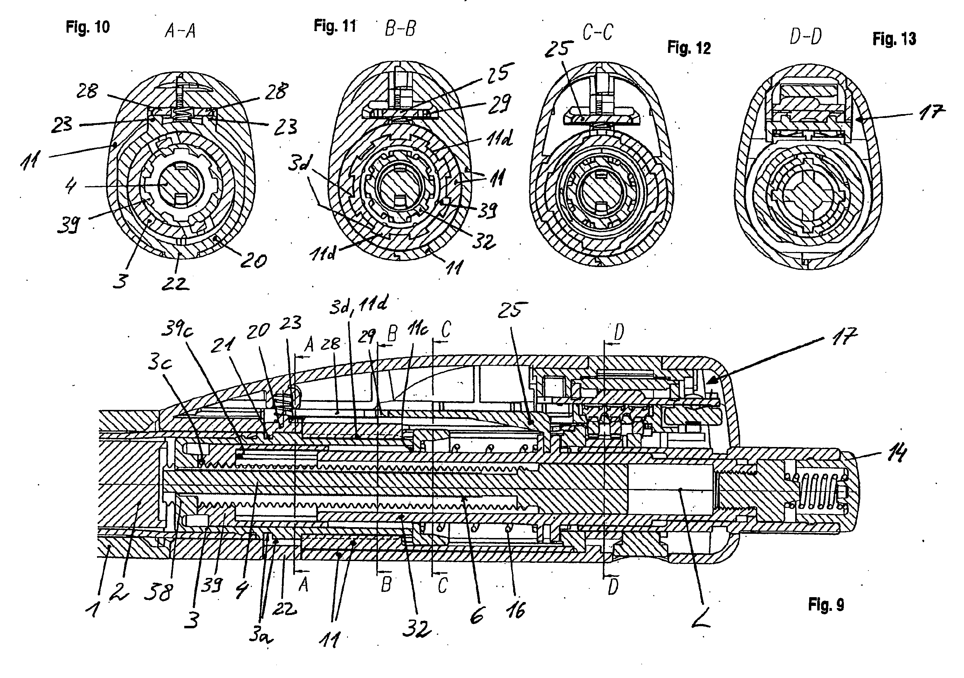

Die Figuren 9 bis 13 zeigen in einem Längsschnitt und vier Querschnitten ein zweites Ausführungsbeispiel eines Injektionsgeräts. Das Injektionsgerät des zweiten Ausführungsbeispiels gleicht demjenigen des ersten Ausführungsbeispiels in Bezug auf die Verriegelung und die Verriegelungssperre 25 derart, dass auf die diesbezügliche Beschreibung des ersten Ausführungsbeispiels verwiesen wird. Insbesondere ist die Verriegelungssperre 25 des zweiten Ausführungsbeispiels in Bezug auf alle funktionalen Details mit derjenigen des ersten Ausführungsbeispiels identisch. Das gleiche gilt für die Verriegelungselemente 3a und 21.

Der Verriegelungsring 20 und die Lage der Sperrnocken 23 relativ zu dem Verriegelungselement 21 und relativ zu der Verriegelungssperre 25 in dem Ausgangszustand des Geräts sind besonders gut in den Querschnitten der Figuren 10, 11 und 12 zu erkennen, auf die diesbezüglich auch stellvertretend für das erste Ausführungsbeispiel verwiesen wird.

Das Injektionsgerät des zweiten Ausführungsbeispiels unterscheidet sich von dem ersten Ausführungsbeispiel durch den Eingriff und den Bewegungsablauf der an der Dosierung beteiligten Komponenten. Ferner erfüllt der Mechanikhalter über die Funktionen des Mechanikhalters des ersten Ausführungsbeispiels hinaus insbesondere die Funktion der Positionierung des Dosiseinstellglieds in diskreten Drehwinkelpositionen, die relativ zu dem Mechanikhalter zum Zwecke der Dosierung veränderbar sind. Die Blockiereinrichtung des zweiten Ausführungsbeispiels ist hingegen einfacher ausgeführt als die des ersten Ausführungsbeispiels. Im Folgenden werden in erster Linie nur die Unterschiede im Vergleich zum ersten Ausführungsbeispiel beschrieben, wobei für Komponenten, die ihrer grundsätzlichen Funktion nach den gleichnamigen Komponenten des ersten Ausführungsbeispiels gleichen, aber in Details unterscheiden, 30iger Zahlen mit gleicher Endziffer oder exakt die gleichen Bezugszeichen, wie bei dem ersten Ausführungsbeispiel, verwendet werden. Soweit zum zweiten Ausführungsbeispiel keine Ausführungen gemacht werden, sollen die entsprechenden Ausführungen zum ersten Ausführungsbeispiel gelten.

In dem zweiten Ausführungsbeispiel ist das relativ zu dem hinteren Gehäuseabschnitt 11 axial linear bewegbare und um die Längsachse L verdrehbare Dosier- und Betätigungselement 32 mit dem Dosiseinstellglied 39 verdrehgesichert verbunden. Das Dosier- und Betätigungselement 32 und das Dosiseinstellglied 39 sind relativ zueinander und relativ zu den Gehäuseabschnitten 1, 3 und 11 in und gegen die Vorschubrichtung bewegbar. Die Kolbenstange 4 wird von dem Mechanikhalter 3 verdrehgesichert gehalten. Die mit dem ersten Ausführungsbeispiel funktionsgleiche Rückzugsperreinrichtung 6 verhindert im Zusammenwirken mit Sperrelementen der einstückig an dem Mechanikhalter 3 geformten Blockiereinrichtung 38 eine Bewegung der Kolbenstange 4 entgegen der Vorschubrichtung, lässt eine Bewegung in die Vorschubrichtung jedoch zu. Die Sperrelemente bilden gleichzeitig die Rückzugsperre und die Verdrehsicherung für die Kolbenstange 4. Des Weiteren bildet wie bereits im ersten Ausführungsbeispiel das Dosier- und Betätigungselement 32 eine Gleitführung für die Kolbenstange 4.

Bei der Dosierung führt das Dosier- und Betätigungselement 32 die gleiche rotatorische Dosierbewegung wie das Dosier- und Betätigungselement 12 des ersten Ausführungsbeispiels aus. Allerdings wird aufgrund des verdrehgesicherten Eingriffs das Dosiseinstellglied 39 bei der rotatorischen Dosierbewegung mitgenommen. Der Gewindeeingriff zwischen der Kolbenstange 4 und dem Dosiseinstellglied 39 ist wieder mit demjenigen des ersten Ausführungsbeispiels vergleichbar, so dass ein von dem Dosiseinstellglied 39 gebildeter Anschlag 39c aufgrund der rotatorischen Dosierbewegung und des Gewindeeingriffs mit der Kolbenstange 4 im Zuge der Dosierung entgegen der Vorschubrichtung auf ein vorderes Ende des Dosier- und Betätigungselements 32 zu bewegt wird. Im Gegensatz zum ersten Ausführungsbeispiel vollführt das Dosiseinstellglied 39 somit bei der Dosierung eine rotatorische Dosierbewegung und eine translatorische Dosierbewegung relativ zu dem vorderen Gehäuseabschnitt, während die Kolbenstange 4 stillsteht. Durch die nach Abschluss der Dosierung erfolgende Ausschüttbewegung des Dosier- und Betätigungselements 32 wird die Kolbenstange 4 um die Weglänge vorgeschoben, die dem durch die Dosierung eingestellten lichten Abstand zwischen einer Anschlagfläche des Dosiseinstellglieds 39 und dem Ausschüttanschlag 3c des Mechanikhalters 3 entspricht.

Die translatorische Dosierbewegung des Dosiseinstellglieds 39 wird gegen die Vorschubrichtung von einem hinteren Translationsanschlag 11c begrenzt, den der hintere Gehäuseabschnitt 11 selbst unmittelbar bildet. Auch bei dem zweiten Ausführungsbeispiel bildet die Längsachse L die Rotations- und Translationsachse der Komponenten, die an der Dosierung und Produktausschüttung beteiligt sind.

Der vordere Gehäuseabschnitt 1, 3 bildet wie bei dem ersten Ausführungsbeispiel eine Gleitführung für das Dosiseinstellglied 39. Zur Ausbildung der Gleitführung stehen eine innere Mantelfläche des Mechanikhalters 3 und eine äußere Mantelfläche des Dosiseinstellglieds 39 miteinander in Gleitkontakt. Das Dosier-und Betätigungselement 32 steht mit einer Innenmantelfläche des Dosiseinstellglieds 39 in einem Eingriff, um die verdrehgesicherte Verbindung zwischen dem Dosiseinstellglied 39 und dem Dosier- und Betätigungselement 32 zu bilden.

In dem zweiten Ausführungsbeispiel weist die Kolbenstange 4 über die Rückzugssperreinrichtung 6 hinaus keine eigene Bremseinrichtung auf. Die Vorderseiten der Sägezähnen der Rückzugssperreinrichtung 6 bilden vielmehr alleine auch die Bremseinrichtung. Die Kolbenstange 4 des zweiten Ausführungsbeispiels kann jedoch durch die Kolbenstange des ersten Ausführungsbeispiels ersetzt werden. Entsprechend wären in diesem Falle durch den Mechanikhalter 3 des zweiten Ausführungsbeispiels auch wenigstens ein Bremselement, vorzugsweise beide Bremselemente des ersten Ausführungsbeispiels auszubilden.

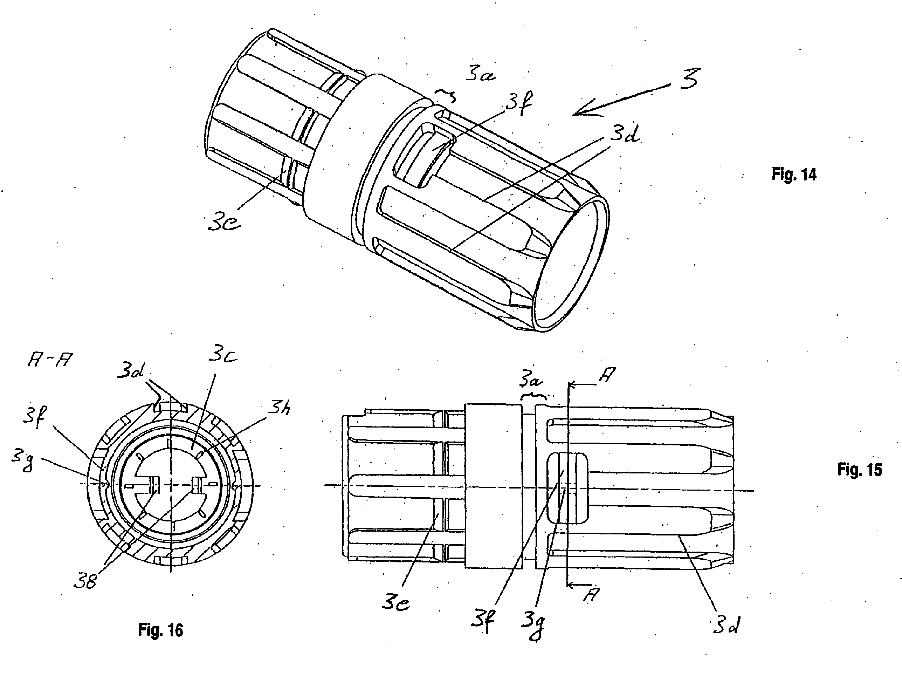

Die Figuren 14 bis 16 zeigen den Mechanikhalter 3 des zweiten Ausführungsbeispiels in einer perspektivischen Darstellung, einer Seitenansicht und in dem in der Seitenansicht eingetragenen Querschnitt A-A. Der Mechanikhalter 3 ist wie beim ersten Ausführungsbeispiel als einteiliges Hülsenteil ausgeführt, vorzugsweise als Kunststoffspritzgussteil. Er weist an dem Außenmantel eines vorderen Hülsenabschnitts einen Wulst 3e auf. Der vordere Hülsenabschnitt wird in das Reservoirteil 1 eingesteckt und mittels des Wulstes 3e zumindest für den Verwender unlösbar mit dem Reservoirteil 1 verrastet.

Das Verriegelungselement 3a ist an einem mittleren Hülsenabschnitt des Mechanikhalters 3 wie bei dem ersten Ausführungsbeispiel ausgebildet.

Ein hinterer Hülsenabschnitt, der sich an das Verriegelungselement 3a anschließt, bildet an seinem Außenumfang eine Mehrzahl von Axialführungen 3d. Die Axialführungen 3d werden von Führungsrippen gebildet, die an dem Außenumfang des hinteren Hülsenabschnitts radial aufragen. Genauer gesagt wird die Axialführung von den axial sich erstreckenden, geraden Seitenwänden dieser Führungsrippen gebildet, so dass, wie bei dem ersten Ausführungsbeispiel, axiale Führungskanäle erhalten werden. Die Führungsrippen ragen von dem mittleren Hülsenabschnitt aus wie Finger bis an das hintere Ende des Mechanikhalters 3, wo sie axial verjüngt auslaufen. Die Axialführung 3d dient der Linearführung des hinteren Gehäuseabschnitts 11 bei dem Verbinden des Reservoirmoduls 10 mit dem Dosier- und Betätigungsmodul 30. Wie in Figur 9 und am besten in Figur 11 zu erkennen ist ragen von einer Innenmantelfläche des hinteren Gehäuseabschnitts 11 der Anzahl nach entsprechend und der Form nach angepasst Eingriffselemente 11 d radial einwärts ab. In jede der Axialführungen 3d ragt ein Eingriffselement 11 d ein und wird von der Axialführung 3d linear geführt, wenn der vordere Gehäuseabschnitt 1, 3 und der hintere Gehäuseabschnitt 11 zum Verbinden ineinander geschoben werden. Auf diese Weise wird sichergestellt, dass zwischen dem vorderen Gehäuseabschnitt 1, 3 und dem hinteren Gehäuseabschnitt 11 keine Relativdrehung stattfinden kann, wenn im Zuge des Verbindens der verdrehgesicherte Eingriff zwischen dem Dosier- und Betätigungselement 32 und dem Dosiseinstellglied 39 hergestellt wird.

Indem die Führungsrippen an ihren hinteren Enden axial verjüngt auslaufen und die Führungskanäle so zu Einführtrichtern verbreitert sind, wird eine Zentrierung zwischen dem vorderen Gehäuseabschnitt 1, 3 und dem hinteren Gehäuseabschnitt 11 zum Zwecke des Verbindens erleichtert. Die Führungsrippen laufen an ihren Enden auch radial zur Mantelfläche des Mechanikhalters 3 verjüngt aus, wodurch die Zentrierung der Gehäuseabschnitte 1, 3 und 11 in eine durch die Axialführung 3d vorgegebene Drehwinkelposition relativ zueinander noch mehr erleichtert wird.

Ebenso wie bei dem Ineinanderschieben der vordere Gehäuseabschnitt 1, 3 und der hintere Gehäuseabschnitt 11 an einer Relativdrehung zueinander gehindert werden, wird auch das Dosiseinstellglied 39 in Bezug auf seine Drehwinkelposition relativ zu dem vorderen Gehäuseabschnitt 1, 3 fixiert, wobei diese Fixierung lösbar ist, um die für die Dosierung erforderliche Drehbewegung des Dosiseinstellglieds 39 zuzulassen. Um daher zum einen die Dosierbewegung des Dosiseinstellglieds 39 zu ermöglichen, aber zum anderen eine unerwünschte Dosierbewegung durch das Herstellen der Verbindung zwischen dem vorderen Gehäuseabschnitt 1, 3 und dem hinteren Gehäuseabschnitt 11 zu verhindern, wird das Dosiseinstellglied 39 von dem Mechanikhalter 3 mittels einer lösbaren Rastverbindung in diskreten Drehwinkelpositionen fixiert.

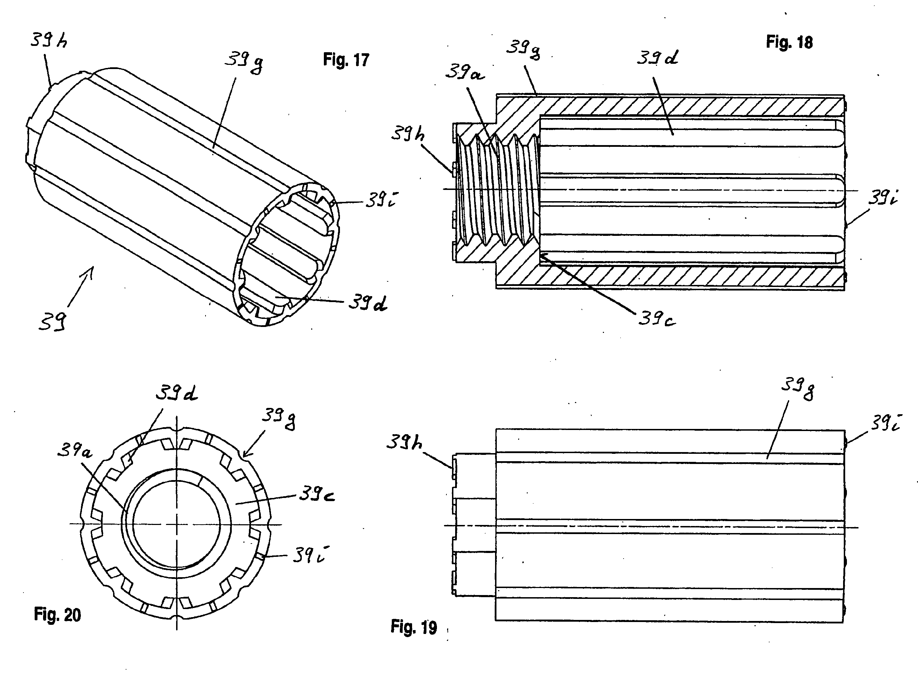

Die Figuren 17 bis 20 zeigen das Dosiseinstellglied 39 in Einzeldarstellungen. Für die Ausbildung der Rastverbindung sind an der Außenmantelfläche des Dosiseinstellglieds 39 über den Umfang in regelmäßiger Teilung verteilt mehrere Rastaufnahmen 39g ausgebildet. Jede der Rastaufnahmen 39g wird von einer geraden, axial sich erstreckenden Rinne mit einer im Querschnitt gerundet verlaufenden Kontur gebildet.

Der Mechanikhalter 3 ist mit zwei Rastnasen 3g (Figur 15 und 16) versehen. Die beiden Rastnasen 3g ragen in dem hinteren Hülsenabschnitt des Mechanikhalters 3 von einer Innenmantelfläche des Mechanikhalters 3 radial einwärts ab. Sie sind einander diametral gegenüberliegend angeordnet. Der jeweilige Mantelbereich des Mechanikhalters 3, an dem eine der Rastnasen 3g angeformt ist, bildet ein in radialer Richtung elastisch nachgiebiges Federelement 3f. Aufgrund der elastischen Nachgiebigkeit und der gerundeten Form der Rastnasen 3g in Verbindung mit dem gerundeten Profil der Rastaufnahmen 39g ist der Rasteingriff der Rastnasen 3g in die gegenüberliegenden Rastaufnahmen 39g lösbar. Die Lösbarkeit ist für die Dosisauswahl erforderlich. Andererseits ist der Rasteingriff jedoch so gestaltet, dass die Drehwinkelfixierung des Dosiseinstellglieds 39 so stabil ist, dass bei dem Verbinden des vorderen Gehäuseabschnitts 1, 3 mit dem hinteren Gehäuseabschnitt 11 keine unerwünschte Dosierbewegung des Dosiseinstellglieds 39 stattfinden kann, wenn die Drehkopplung des Dosier- und Betätigungselements 32 mit dem Dosier- und Betätigungselement 32 hergestellt wird. Die Rastverbindung zwischen dem Mechanikhalter 3 und dem Dosiseinstellglied 39 ergibt als vorteilhaften Nebeneffekt ein taktiles Signal bei der Dosierung. Um die günstige Elastizität des Federelements 3f zu erhalten, ist der hintere Hülsenabschnitt des Mechanikhalters 3 im betreffenden Mantelbereich ausgeschnitten, so dass das Federelement 3f als ein im Umfangsrichtung sich erstreckendes Ringsegment erhalten wird, das axial beidseits freiliegt.

Aus den Figuren 17, 18 und 20 ebenfalls erkennbar sind Axialführungen 39d für den verdrehsicheren Eingriff des Dosiseinstellglieds 39 mit dem Dosier- und Betätigungselement 32. Das Dosier- und Betätigungselement 32 ist mit wenigstens einem Eingriffselement versehen, um die axiale Linearführung, d.h. die Verdrehsicherung, zwischen dem Dosier- und Betätigungselement 32 und dem Dosiseinstellglied 39 zu erhalten. Die Axialführungen 39d sind wieder Führungskanäle, die von mehreren, axial sich gerade erstreckenden Führungsrippen gebildet werden. Jede der Führungsrippen läuft an ihrem hinteren, dem Dosier- und Betätigungselement 32 zugewandten Ende axial und radial verjüngt aus, um die Zentrierung zwischen dem Dosier- und Betätigungselement 32 und dem Dosiseinstellglied 39 bei der Herstellung des verdrehsicheren Eingriffs zu erleichtern. Für die axiale Linearführung von Dosiseinstellglied 39 und Dosier-und Betätigungselement 32 wird somit die gleiche Konstruktion wie für die axiale Linearführung der Gehäuseabschnitte 1, 3 und 11 verwendet.

Der Vollständigkeit wegen sei schließlich auch auf das Dosiergewinde 39a und den Ausschüttanschlag 39c des Dosiseinstellglieds 39 hingewiesen, die am besten in Figur 18 zu erkennen sind.

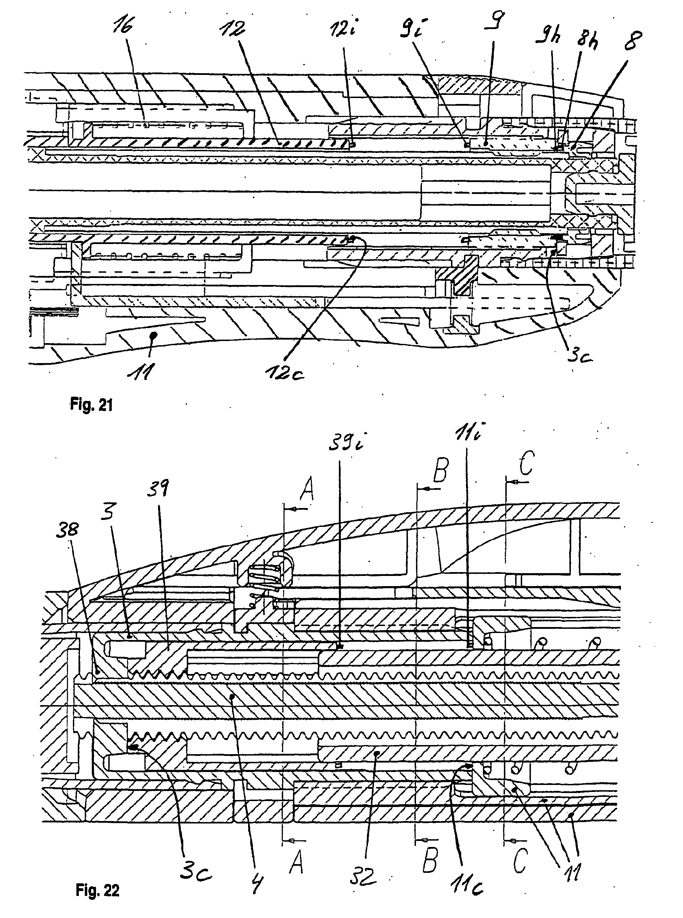

Schließlich sind für das Dosiseinstellglied 39 zwei Verdrehsicherungen vorgesehen, die in den beiden axialen Endpositionen des Dosiseinstellglieds 39 wirksam werden. Diesbezüglich sei ergänzend auf Figur 22 hingewiesen.

Um zu verhindern, dass die Kolbenstange 4 in Reaktion auf eine rotatorische Dosierbewegung des Dosiseinstellglieds 39 zurückbewegt werden könnte, sind an einem vorderen Ende des Dosiseinstellglieds 39 Verdrehanschläge 39h geformt. Die Verdrehanschläge 39h stehen in der vorderen Endposition, die das Dosiseinstellglied 39 unmittelbar nach einer Produktausschüttung bzw. vor einer Dosisauswahl einnimmt, mit Verdrehgegenanschlägen 3h in Eingriff, die an dem Mechanikhalter 3 angeformt sind (Fig. 16). Die Verdrehanschläge 39h ragen von einer vorderen Stirnseite des Dosiseinstellglieds 39 axial ab, und die Verdrehgegenanschläge 3h ragen von einer axial zugewandten Stirnfläche des Mechanikhalters 3, die den Aufschüttanschlag 3c bildet, den Verdrehanschlägen 39h axial entgegen. Der Eingriff zwischen den Verdrehanschlägen 39h und den Verdrehgegenanschlägen 3h ist derart, dass er eine rotatorische Dosierbewegung in eine Drehrichtung zulässt, die eine von dem Ausschüttanschlag 3c weg gerichtete translatorische Dosierbewegung des Dosiseinstellglieds 39 bewirkt, aber in der vorderen axialen Endposition eine rotatorische Dosierbewegung in die Gegendrehrichtung verhindert.

Ferner ist ein weiteres Paar von Verdrehanschlägen und Verdrehgegenanschlägen vorgesehen, die in grundsätzlich gleicher Weise wie die Anschläge 3h und 39h geformt sind und zusammenwirken. Bei diesem zweiten Verdrehanschlagpaar handelt es sich zum einen um Verdrehanschläge 39i, die von einer hinteren Stirnfläche des Dosiseinstellglieds 39 axial abragen, und zum anderen um Verdrehgegenanschläge 11 i, die von der zugewandten Anschlagstirnfläche des hinteren Translationsanschlags 11 c axial auf das Dosiseinstellglied 39 zuragen, in der Figur 9 aufgrund ihrer geringen Abmessungen jedoch nicht erkennbar sind. Durch das hintere Verdrehanschlagpaar 11i/39i wird in der hinteren Endposition verhindert, dass die Kolbenstange 4 in Reaktion auf eine gegen die Anschlagstirnfläche des hinteren Translationsanschlags 11 c gerichtete Dosierbewegung des Dosiseinstellglieds in Vorschubrichtung bewegt werden kann.

Die Höhe, d.h. die axiale Länge, sämtlicher Verdrehanschläge 3h, 39h, 11 i und 39i ist auf die Gewindesteigung der im Eingriff befindlichen Dosiergewinde der Kolbenstange 4 und des Dosiseinstellglieds 39 abgestimmt. Die Verdrehanschläge sind axial so kurz, dass diejenige rotatorische Dosierbewegung nicht behindert wird, durch die das Dosiseinstellglied 39 von dem jeweiligen Translationsanschlag 3c oder 11 c wegbewegt wird.

Bei der Montage der Komponenten des Reservoirmoduls 10 wird das Dosiseinstellglied 39 bis in eine vorgegebene Axialposition, wie sie aus Figur 9 ersichtlich ist, auf die Kolbenstange 4 aufgeschraubt. Anschließend wird die Kolbenstange 4 mit dem aufgeschraubten Dosiseinstellglied 39 von hinten in den Mechanikhalter 3 eingeführt, bis dessen Blockiereinrichtung 38 in Sperreingriff mit der Rückzugssperreinrichtung 6 der Kolbenstange 4 gelangt und ferner der verdrehgesicherte Eingriff zwischen den Verdrehanschlägen 39h des Dosiseinstellglieds 39 und den Verdrehgegenanschlägen 3h des Mechanikhalters 3 hergestellt ist. Bereits während der Einführung in den Mechanikhalter 3 wird das Dosiseinstellglied 39 in einer Drehwinkelrastposition durch den Rasteingriff zwischen den Rastnasen 3g und den Rastaufnahmen 39g von dem Mechanikhalter 3 axial linear geführt, bis das Dosiseinstellglied 39 an den Ausschüttanschlag 3c des Mechanikhalters 3 anstößt. In dieser vorderen Endposition des Dosiseinstellglieds 39 relativ zu dem Mechanikhalter 3 ist gleichzeitig auch bereits der verdrehsichere Eingriff zwischen den Verdrehanschlägen 3h und 39h hergestellt.

In diesem Zustand werden der Mechanikhalter 3 und ein bereits mit einem Reservoir ausgestattetes Reservoirteil 1 miteinander verbunden.

In einem nächsten Schritt wird der hintere Gehäuseabschnitt 11 des komplett montierten Dosier- und Betätigungsmoduls 30 auf den Mechanikhalter 3 geschoben, wobei sich der Mechanikhalter 3 und der hintere Gehäuseabschnitt 11 aufgrund der Axialführungen 3d und der Eingriffselemente 11 d des hinteren Gehäuseabschnitts 11 zueinander zentrieren können und nach der Zentrierung aufgrund des Führungseingriffs axial aneinander linear geführt werden. Im Zuge des Aufschiebens gelangt das Dosier- und Betätigungselement 32 in den verdrehgesicherten Eingriff mit dem Dosiseinstellglied 39, wobei auch hier durch eine den Axialführungen 3d und Eingriffselementen 11 d entsprechende Linearführung zuerst eine gewisse Zentrierung stattfinden kann.

Das Dosier- und Betätigungselement 32 ist in diskreten Drehwinkelrastpositionen mit dem hinteren Gehäuseabschnitt in einem Rasteingriff und wird in dem Rasteingriff, d.h. in der jeweiligen Drehwinkelrastposition, axial linear geführt. Die Drehwinkeldifferenz zwischen zwei aufeinanderfolgenden Drehwinkelrastpositionen entspricht einer Dosiseinheit. Die Linearführung zwischen dem Mechanikhalter 3 und dem hinteren Gehäuseabschnitt 11 einerseits und die diskreten Drehwinkelpositionen des Dosiseinstellglieds 39 relativ zu dem Mechanikhalter 3 (Rastnasen 3g und Rastaufnahmen 39g) und die Drehwinkelrastpositionen des Dosier- und Betätigungselements 32 relativ zu dem hinteren Gehäuseabschnitt 11 andererseits sind aufeinander so abgestimmt, dass ein lineares Übereinanderschieben der beiden Gehäuseabschnitte 1, 3 und 11 stets in solch einer Drehwinkelposition stattfindet, dass auch das Dosiseinstellglied 39 und das Dosier- und Betätigungselement 32 für ihren verdrehgesicherten Eingriff relativ zueinander ausgerichtet sind, so dass während des Verbindens des Reservoirmoduls 10 mit dem Dosier- und Betätigungsmodul 30 keine Relativdrehung zwischen den an der Dosierung beteiligten Komponenten stattfindet.

In Bezug auf die weiteren Details der Montage, insbesondere der Herstellung des Verriegelungseingriffs, und zur Funktionsweise des Injektionsgeräts nach dem zweiten Ausführungsbeispiel wird auf die Beschreibung zu dem ersten Ausführungsbeispiel verwiesen.

Auch bei dem Injekionsgerät nach dem ersten Ausführungsbeispiel können Verdrehsicherungen vorgesehen sein, die in den beiden axialen Endpositionen des Dosiseinstellglieds 9 des ersten Ausführungsbeispiels unerwünschte Reaktionsbewegungen der Kolbenstange 4 verhindern. Figur 21 zeigt die beiden Verdrehsicherungen, die in gleicher Weise wie die Verdrehsicherungen des zweiten Ausführungsbeispiels geformt sind. Allerdings werden die bei dem zweiten Ausführungsbeispiel an den Gehäuseabschnitten 1, 3 und 11 geformten Verdrehgegenanschläge bei dem ersten Ausführungsbeispiel zum einen von der Blockiereinrichtung 8 und zum anderen von dem Dosier- und Betätigungselement 12 gebildet. So sind an der Stirnfläche der Blockiereinrichtung 8, die dem Dosiseinstellglied 9 axial zugewandt ist, mehrere Verdrehanschläge 8h angeformt, die auf das Dosiseinstellglied 9 axial zu ragen. Da die Blockiereinrichtung 8 von dem vorderen Gehäuseabschnitt 1, 3 axial unbeweglich gelagert wird und mit der Kolbenstange 4 verdrehgesichert verbunden ist, wird auch durch das vordere Verdrehanschlagpaar 8h/9h eine Verdrehsicherung für die zwischen der Kolbenstange 4 und dem Dosiseinstellglied 9 stattfindende rotatorische Dosierbewegung erhalten. Das zweite Verdrehanschlagpaar ist zwischen dem Dosiseinstellglied 9 und dem hinteren Translationsanschlag 12c gebildet. Wie bei dem zweiten Ausführungsbeispiel ragen von der Stirnfläche des Translationsanschlags 12c, die dem Dosiseinstellglied 9 axial zugewandt ist, mehrere Verdrehanschläge 12i axial in Richtung auf das Dosiseinstellglied 9 vor. Das Dosiseinstellglied 9 ist wie bei dem zweiten Ausführungsbeispiel an seiner Rückseite mit Verdrehanschlägen 9i versehen, die in der hinteren axialen Endposition des Dosiseinstellglieds 9 in Eingriff mit den Verdrehanschlägen 12i gelangen. In der hinteren axialen Endposition des Dosiseinstellglieds 9 wird durch das hintere Verdrehanschlagpaar 9i/12i nur diejenige rotatorische Dosierbewegung zugelassen, die eine translatorische Dosierbewegung des Dosiseinstellglieds 9 in die Vorschubrichtung bewirkt.

### Bezugszeichen:

- 1: Reservoirteil, Ampullenhalter
- 2: Reservoir, Ampulle
- 3: Mechanikhalter
- 3a: Erstes Verriegelungselement
- 3b: Fixiereinrichtung
- 3c: Ausschüttanschlag, Translationsanschlag
- 3d: Axialführung
- 3e: Wulst
- 3f: Federelement
- 3g: Rastnase
- 3h: Verdrehanschlag
- 4: Kolbenstange
- 4a: Verbindungsabschnitt
- 5: Gewindeabschnitt
- 6: Rückzugsperreinrichtung, Zahnreihe
- 7: Vorschubbremseinrichtung, Zahnreihe
- 8: Blockiereinrichtung
- 8a: Sperrelement
- 8b: Bremselement
- 8h: Verdrehanschlag
- 9: Dosiseinstellglied
- 9h: Verdrehanschlag
- 9i: Verdrehanschlag
- 10: Reservoirmodul
- 11: hinterer Gehäuseabschnitt
- 11d: Eingriffselement
- 11i: Verdrehanschlag
- 12: Dosier- und Betätigungselement
- 12i: Verdrehanschlag
- 13: Dosiermitnehmer
- 14: Verschluss
- 15a: Mitnehmer, Ringsteg
- 15b: Mitnehmer, Ringsteg
- 16: Rückstelleinrichtung
- 17: Zähl- und Anzeigeeinrichtung
- 18: -
- 19: -
- 20: Verriegelungsring
- 21: Zweites Verriegelungselement
- 22: Entriegelungsknopf
- 23: Sperrnocken
- 24: Rückstelleinrichtung
- 25: Verriegelungssperre
- 26: Mitnehmer, Steg
- 27: Axialausnehmung
- 28: Sperrzunge
- 29: Einrückausnehmung
- 30: Dosier- und Betätigungsmodul
- 31: Anschlagelement
- 32: Dosier- und Betätigungselement
- 33-37: -
- 38: Blockiereinrichtung
- 39: Dosiseinstellglied
- 39a: Dosiergewinde
- 39c: Ausschüttanschlag
- 39d: Axialführung
- 39g: Rastaufnahme, Axialführung
- 39h: Verdrehanschlag
- 39i: Verdrehanschlag

## Patentansprüche

1. Verabreichungsgerät mit Dosiervorrichtung, das Verabreichungsgerät umfassend:
a) ein Gehäuse (1,3, 11) mit einem Reservoir (2) für ein ausschüttbares Produkt,
b) einen Kolben, der in dem Reservoir (2) auf einen Reservoirauslass zu bewegbar ist, um Produkt auszuschütten,
c) eine Kolbenstange (4),
d) ein Antriebselement (12), mit dem relativ zu dem Gehäuse (1,3, 11) eine Drehbewegung vorgenommen werden kann,
**dadurch gekennzeichnet, dass**
d1) das Antriebselement ein Dosier- und Antriebselement (12) ist, mit dem relativ zu dem Gehäuse (1, 3, 11) eine Dosierbewegung in der Form einer Drehbewegung zur Auswahl einer Produktdosis und eine Ausschüttbewegung in der Form einer Linearbewegung zur Ausschüttung der Produktdosis ausführbar sind, und das mit der Kolbenstange (4) in einem Eingriff ist, der eine Mitnahme der Kolbenstange (4) bei der Drehbewegung bewirkt, aber die lineare Ausschüttbewegung des Dosier- und Antriebselements (12) relativ zu der Kolbenstange (4) zulässt,
e) und ein Dosiseinstellglied (9), das von dem Dosier- und Antriebselement (12) bei der Ausschüttbewegung in die Vorschubrichtung bewegt wird und das mit der Kolbenstange (4) und dem Gehäuse (1,3, 11) je in solch einem Eingriff steht, dass es nur gemeinsam mit der Kolbenstange (4) in die Vorschubrichtung bewegbar ist und durch die Dosierbewegung relativ zu der Kolbenstange (4) entgegen der Vorschubrichtung bewegt wird.

2. Verabreichungsgerät nach dem vorhergehenden Anspruch, **dadurch gekennzeichnet, dass** das Dosiseinstellglied (9) und das Dosier- und Antriebselement (12) in Bezug auf die Vorschubrichtung überlappungsfrei hintereinander angeordnet sind.

3. Verabreichungsgerät nach einem der vorhergehenden Ansprüche, **dadurch gekennzeichnet, dass** eine Blockiereinrichtung(8) vorgesehen ist, die mit der Kolbenstange (4) in Eingriff steht, um eine Bewegung der Kolbenstange (4) entgegen der Vorschubrichtung zu verhindern.

4. Verabreichungsgerät nach einem der vorhergehenden Ansprüche, **dadurch gekennzeichnet, dass** eine Blockiereinrichtung (8) vorgesehen ist, die mit der Kolbenstange (4) in einem Bremseingriff steht, der die Bewegung der Kolbenstange (4) in die Vorschubrichtung erschwert.

5. Verabreichungsgerät nach einem der zwei vorhergehenden Ansprüche, **dadurch gekennzeichnet, dass** die Kolbenstange (4) und die Blockiereinrichtung (8) in solch einem Eingriff stehen, dass die Kolbenstange (4) bei der Dosierbewegung die Blockiereinrichtung (8) mitnimmt.

6. Verabreichungsgerät nach einem der drei vorhergehenden Ansprüche, **dadurch gekennzeichnet, dass** die Blockiereinrichtung (8) um eine Längsachse der Kolbenstange (4) drehbar von dem Gehäuse (1,3, 11) gelagert wird.

7. Verabreichungsgerät nach einem der vier vorhergehenden Ansprüche, **dadurch gekennzeichnet, dass** die Kolbenstange (4) wenigstens eine Zahnreihe (6; 7) aufweist, in die wenigstens ein Eingriffselement (8a; 8b) der Blockiereinrichtung (8) eingreift.

8. Verabreichungsgerät nach einem der vorhergehenden Ansprüche, **dadurch gekennzeichnet, dass** das Gehäuse (1,3, 11) eine Linearführung für das Dosiseinstellglied (9) bildet, in die das Dosiseinstellglied (9) eingreift.

9. Verabreichungsgerät nach dem vorhergehenden Anspruch, **dadurch gekennzeichnet, dass** das Gehäuse (1,3, 11) wenigstens einen vorderen Gehäuseabschnitt (1,3) und einen hinteren Gehäuseabschnitt (11.) umfasst, die lösbar miteinander verbunden sind, der vordere Gehäuseabschnitt (1,3) das Reservoir (2) enthält und die Linearführung für das Dosiseinstellglied (9) bildet, und der hintere Gehäuseabschnitt (11) das Dosier-und Antriebselement (12) umfasst.

10. Verabreichungsgerät nach einem der vorhergehenden Ansprüche, **dadurch gekennzeichnet, dass** eine Kanüle von höchstens 30 Gauge, vorzugsweise eine 31 oder 32 Gauge Kanüle, oder eine Kanüle, die eine nicht in ISO 9626 spezifizierte Kombination von Aussendurchmesser und Innendurchmesser hat, mit einem Aussendurchmesser von höchstens 320 µm ein infundierendes Teil des Injektionsgeräts bildet.

11. Reservoirmodul für ein Verabreichungsgerät nach einem der vorhergehenden Ansprüche, **dadurch gekennzeichnet, dass** das Reservoirmodul umfasst:
a) einen vorderen Gehäuseabschnitt (1,3) des Verabreichungsgeräts, der ein Reservoir (2) für ein ausschüttbares Produkt und eine Verbindungseinrichtung (3d, 20,21) für eine Verbindung mit einem hinteren Gehäuseabschnitt des Verabreichungsgeräts aufweist,
b) einen Kolben, der in dem Reservoir (2) in eine Vorschubrichtung auf einen Reservoirauslass zu verschiebbar aufgenommen ist, um Produkt auszuschütten,
c) eine Kolbenstange (4), die von dem vorderen Gehäuseabschnitt (1,3) um ihre Längsachse (L) drehbar gelagert wird und ein Gewinde aufweist,
d) und ein Dosiseinstellglied (9), das mit der Kolbenstange (4) in einem Gewindeeingriff steht und von dem vorderen Gehäuseabschnitt (1,3) so geführt wird, dass eine Drehbewegung der Kolbenstange (4) um ihre Längsachse eine Bewegung des Dosiseinstellglieds (9) entgegen der Vorschubrichtung bewirkt, e) wobei die Kolbenstange (4) einen Verbindungsabschnitt (4a) für eine Kopplung mit einem Dosier-und Antriebselement des Verabreichungsgeräts aufweist und wobei der Verbindungsabschnitt (4a) so ausgebildet ist, dass die Kopplung der Kolbenstange (4) mit dem Dosier- und Antriebselement des Verabreichungsgeräts eine Relativverschiebung zwischen dem Dosier- und Antriebselement und der Kolbenstange (4) entlang der Längsachse (L) der Kolbenstange (4) zulässt und eine Relativdrehung zwischen dem Dosier-und Antriebselement und der Kolbenstange (4) um die Längsachse (L) der Kolbenstange (4) verhindert.

12. Reservoirmodul nach dem vorhergehenden Anspruch, **dadurch gekennzeichnet, dass** der vordere Gehäuseabschnitt (1,3) ein hülsenförmiges Reservoirteil (1) mit dem Reservoir (2) und einen hülsenförmigen Mechanikhalter (3) umfasst, die separat gefertigt und lösbar oder unlösbar miteinander verbunden sind, wobei der Mechanikhalter (3) die Kolbenstange (4) hält und das Dosiseinstellglied (9) führt.

13. Reservoirmodul nach einem der vorhergehenden Ansprüche, **dadurch gekennzeichnet, dass** das Reservoirmodul (10) ein Verbrauchsmodul ist, das nach einer Entleerung des Reservoirs (2) für einen Austausch im Ganzen vorgesehen ist.

## Claims

1. An administration device with a metering arrangement, which administration device comprises:
a) a housing (1, 3, 11) with a reservoir (2) for a product which can be expelled,
b) a piston which is movable in the reservoir (2) towards a reservoir outlet to expel product,
c) a piston rod (4), and
d) a drive element (12) with which a rotary movement can be effected relative to the housing (1, 3, 11),
**characterised in that**
d1) the drive element is a metering and drive element (12) with which a metering movement in the form of a rotary movement for the selection of a product dose and an expulsion movement in the form of a linear movement for expelling the product dose can be effected relative to the housing (1, 3, 11), which metering and drive element is in an engagement with the piston rod (4) which provides for entrainment of the piston rod (4) in the rotary movement but allows the linear expulsion movement of the metering and drive element (12) relative to the piston rod (4), and
e) a dose setting member (9) which is moved by the metering and drive element (12) in the expulsion movement in the advance direction and which is in such an engagement with the piston rod (4) and the housing (1, 3, 11) that it is only movable jointly with the piston rod (4) in the advance direction and is moved by the metering movement relative to the piston rod (4) in opposite relationship to the advance direction.

2. An administration device according to the preceding claim **characterised in that** the dose setting member (9) and the metering and drive element (12) are arranged in overlap-free successive arrangement in relation to the advance direction.

3. An administration device according to one of the preceding claims **characterised in that** there is provided a blocking device (8) which is in engagement with the piston rod (4) to prevent a movement of the piston rod (4) in opposite relationship to the advance direction.

4. An administration device according to one of the preceding claims **characterised in that** there is provided a blocking device (8) which is in a braking engagement with the piston rod (4), which makes it difficult for the piston rod (4) to move in the advance direction.

5. An administration device according to one of the two preceding claims **characterised in that** the piston rod (4) and the blocking device (8) are in such an engagement that the piston rod (4) entrains the blocking device (8) in the metering movement.

6. An administration device according to one of the three preceding claims **characterised in that** the blocking device (8) is supported by the housing (1, 3, 11) rotatably about a longitudinal axis of the piston rod (4).

7. An administration device according to one of the four preceding claims **characterised in that** the piston rod (4) has at least one row of teeth (6; 7) into which at least one engagement element (8a; 8b) of the blocking device (8) engages.

8. An administration device according to one of the preceding claims **characterised in that** the housing (1, 3, 11) forms a linear guide for the dose setting member (9), into which guide the dose setting member (9) engages.

9. An administration device according to the preceding claim **characterised in that** the housing (1, 3, 11) includes at least a front housing portion (1, 3) and a rear housing portion (11) which are releasably connected together, the front housing portion (1, 3) contains the reservoir (2) and forms the linear guide for the dose setting member (9) and the rear housing portion (11) comprises the metering and drive element (12).

10. An administration device according to one of the preceding claims **characterised in that** a cannula of at most 30 gauge, preferably a 31 or 32 gauge cannula, or a cannula which is of a combination of outside diameter and inside diameter not specified in ISO 9626, having an outside diameter of at most 320 µm, forms an infusion part of the injection instrument.

11. A reservoir module for an administration device according to one of the preceding claims **characterised in that** the reservoir module comprises:
a) a front housing portion (1, 3) of the administration device, which has a reservoir (2) for a product which can be expelled and a connecting device (3d, 20, 21) for a connection to a rear housing portion of the administration device,
b) a piston which is accommodated in the reservoir (2) slidably in an advance direction towards a reservoir outlet in order to expel product,
c) a piston rod (4) which is supported by the front housing portion (1, 3) rotatably about its longitudinal axis (L) and has a screwthread, and
d) a dose setting member (9) which is in screwthreaded engagement with the piston rod (4) and is so guided by the front housing portion (1, 3) that a rotary movement of the piston rod (4) about its longitudinal axis causes a movement of the dose setting member (9) in opposite relationship to the advance direction, e) wherein the piston rod (4) has a connecting portion (4a) for coupling to a metering and drive element of the administration device and wherein the connecting portion (4a) is so designed that the coupling of the piston rod (4) to the metering and drive element of the administration device permits a relative displacement between the metering and drive element and the piston rod (4) along the longitudinal axis (L) of the piston rod (4) and prevents a relative rotary movement between the metering and drive element and the piston rod (4) about the longitudinal axis (L) of the piston rod (4).

12. A reservoir module according to the preceding claim **characterised in that** the front housing portion (1, 3) includes a sleeve-shaped reservoir portion (1) with the reservoir (2) and a sleeve-shaped mechanism holder (3) which are produced separately and are releasably or non-releasably connected together, wherein the mechanism holder (3) holds the piston rod (4) and guides the dose setting member (9).

13. A reservoir module according to one of the preceding claims **characterised in that** the reservoir module (10) is a disposable module which after emptying of the reservoir (2) is intended for replacement in its entirety.

## Revendications

1. Dispositif d'administration comportant un dispositif de dosage, le dispositif d'administration comprenant :
a) un boîtier (1, 3, 11) équipé d'un réservoir (2) pour un produit pouvant être évacué,
b) un piston qui peut être déplacé dans le réservoir (2) en direction d'une sortie du réservoir pour évacuer le produit,
c) une tige du piston (4),
d) un élément d'entraînement (12) avec lequel un mouvement de rotation peut être exécuté par rapport au boîtier (1, 3, 11),
**caractérisé en ce que**
d1) l'élément d'entraînement est un élément de dosage et d'entraînement (12), avec lequel un mouvement de dosage sous la forme d'un mouvement de rotation afin de choisir une dose de produit, et un mouvement d'évacuation sous la forme d'un mouvement linéaire pour évacuer la dose de produit peuvent être réalisés par rapport au boîtier (1, 3, 11), et qui est engagé sur la tige du piston (4) de manière à provoquer un entraînement de la tige du piston (4) lors du mouvement de rotation, mais qui autorise le mouvement linéaire d'évacuation de l'élément de dosage et d'entraînement (12) par rapport à la tige du piston (4),
e) et par un organe de réglage de la dose (9), qui est déplacé dans la direction d'avancement par l'élément de dosage et d'entraînement (12) lors du mouvement d'évacuation et qui est engagé sur la tige du piston (4) et dans le boîtier (1, 3, 11) de manière à ne pouvoir être déplacé dans la direction d'avancement qu'avec la tige du piston (4) et qui est déplacé par le mouvement de dosage par rapport à la tige du piston (4) dans le sens opposé à la direction d'avancement.

2. Dispositif d'administration selon l'une des revendications précédentes, **caractérisé en ce que** l'organe de réglage de la dose (9) et l'élément de dosage et d'entraînement (12) sont agencés l'un derrière l'autre par rapport à la direction d'avancement, sans se chevaucher.

3. Dispositif d'administration selon l'une des revendications précédentes, **caractérisé en ce qu'**un dispositif de blocage (8) est prévu, qui est engagé sur la tige du piston (4), afin d'empêcher un mouvement de la tige du piston (4) dans le sens opposé à la direction d'avancement.

4. Dispositif d'administration selon l'une des revendications précédentes, **caractérisé en ce qu'**un dispositif de blocage (8) est prévu, qui se trouve en engagement freinant avec la tige du piston (4), ce qui entrave le mouvement de la tige du piston (4) dans la direction d'avancement.

5. Dispositif d'administration selon l'une des deux revendications précédentes, **caractérisé en ce que** la tige du piston (4) et le dispositif de blocage (8) sont engagés de manière à ce que la tige du piston (4) entraîne le dispositif de blocage (8) lors du mouvement de dosage.

6. Dispositif d'administration selon l'une des trois revendications précédentes, **caractérisé en ce que** le dispositif de blocage (8) est monté tournant par rapport au boîtier (1, 3, 11) autour d'un axe longitudinal de la tige du piston (4).

7. Dispositif d'administration selon l'une des quatre revendications précédentes, **caractérisé en ce que** la tige du piston (4) comporte au moins une rangée de dents (6, 7) dans laquelle s'engage au moins un élément d'engagement (8a, 8b) du dispositif de blocage (8).

8. Dispositif d'administration selon l'une des revendications précédentes, **caractérisé en ce que** le boîtier (1,3, 11) forme un guidage linéaire pour l'organe de réglage de la dose (9), dans lequel s'engage l'organe de réglage de la dose (9).

9. Dispositif d'administration selon l'une des revendications précédentes, **caractérisé en ce que** le boîtier (1, 3, 11) comprend au moins une partie avant du boîtier (1, 3) et une partie arrière du boîtier (11), qui sont reliées l'une à l'autre de manière amovible, la partie avant du boîtier (1, 3) contenant le réservoir (2) et formant le guidage linéaire pour l'organe de réglage de la dose (9), et la partie arrière du boîtier (11) comprenant l'élément de dosage et d'entraînement (12).

10. Dispositif d'administration selon l'une des revendications précédentes, **caractérisé en ce qu'**une canule d'au moins 30 gauges, de préférence une canule de 31 ou 32 gauges, ou une canule qui ne possède pas une combinaison de diamètre externe et de diamètre interne spécifiée dans la norme ISO 9626, et dont le diamètre externe est de tout au plus 320 µm, forme une partie d'injection du dispositif d'injection.

11. Module de réservoir pour un dispositif d'administration selon l'une des revendications précédentes, **caractérisé en ce que** le module de réservoir comprend :
a) une partie avant du boîtier (1, 3) du dispositif d'administration, qui comporte un réservoir (2) pour un produit pouvant être évacué et un dispositif de liaison (3d, 20, 21) pour une liaison avec une partie arrière du boîtier du dispositif d'administration,
b) un piston qui est logé dans le réservoir (2) de manière à pouvoir être déplacé, dans une direction d'avancement, vers une sortie du réservoir, pour évacuer le produit,
c) une tige du piston (4) qui est montée tournant autour de son axe longitudinal (L) par rapport à la partie avant du boîtier (1, 3) et qui comporte un filetage,
d) et un organe de réglage de la dose (9) qui est engagé par filetage sur la tige du piston (4) et qui est guidé par la partie avant du boîtier (1, 3), de sorte qu'un mouvement de rotation de la tige du piston (4) autour de son axe longitudinal provoque un mouvement de l'organe de réglage de la dose (9) allant dans le sens opposé à la direction d'avancement, e) la tige du piston (4) comportant une partie de liaison (4a) pour un accouplement avec un élément de dosage et d'entraînement du dispositif d'administration et la partie de liaison (4a) étant conçue de telle sorte que l'accouplement de la tige du piston (4) avec l'élément de dosage et d'entraînement du dispositif d'administration autorise un déplacement relatif entre l'élément de dosage et d'entraînement et la tige du piston (4) et empêche une rotation relative entre l'élément de dosage et d'entraînement et la tige du piston (4) autour de l'axe longitudinal (L) de la tige du piston (4).

12. Module de réservoir selon l'une des revendications précédentes, **caractérisé en ce que** la partie avant du boîtier (1, 3) comporte une partie de réservoir en forme de manchon (1) équipée du réservoir (2) et d'un support de mécanisme en forme de manchon (3), qui sont fabriqués séparément et reliés l'un à l'autre de manière amovible ou inamovible, le support de mécanisme (3) maintenant la tige du piston (4) et guidant l'organe de réglage de la dose (9).

13. Module de réservoir selon l'une des revendications précédentes, **caractérisé en ce que** le module de réservoir (10) est un module consommable qui est prévu pour être entièrement échangé une fois le réservoir (2) vidé.
